(19) Europäisches Patentamt
European Patent Office
Office européen des brevets

(11) EP 4 108 767 A1

(12) EUROPEAN PATENT APPLICATION

(43) Date of publication:
**28.12.2022 Bulletin 2022/52**

(21) Application number: **21180900.9**

(22) Date of filing: **22.06.2021**

(51) International Patent Classification (IPC):
***C12N 9/22*** *(2006.01)* ***C11D 3/386*** *(2006.01)*

(52) Cooperative Patent Classification (CPC):
**C12N 9/22; C11D 3/38636; C11D 3/38681**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **The Procter & Gamble Company**
**Cincinnati, OH 45202 (US)**

(72) Inventors:
- **GORI, Klaus**
  **2880 Bagsvaerd (DK)**
- **NOERGAARD, Allan**
  **2880 Bagsvaerd (DK)**
- **SALOMON, Jesper**
  **2880 Bagsvaerd (DK)**

(74) Representative: **P&G Patent Belgium UK**
**N.V. Procter & Gamble Services Company S.A.**
**Temselaan 100**
**1853 Strombeek-Bever (BE)**

Remarks:
The complete document including Reference Table(s) and the Sequence Listing(s) can be downloaded from the EPO website

# (54) CLEANING OR TREATMENT COMPOSITIONS CONTAINING NUCLEASE ENZYMES

(57) Cleaning or treatment compositions comprising DNase variants and cleaning adjunct. Methods of treating surfaces such as fabrics by contacting the surface with an aqueous wash liquor having the cleaning composition therein. The compositions and methods are particularly for improving whiteness of a fabric, improved soil removal from a fabric, for malodour removal from a fabric, for anti-wrinkle benefits, anti-redeposition benefits and/or for improved drying of a fabric.

EP 4 108 767 A1

## Description

REFERENCE TO A SEQUENCE LISTING

This application contains a Sequence Listing in computer readable form. The computer readable form is incorporated herein by reference.

FIELD OF THE INVENTION

The present invention relates to detergent compositions and methods of cleaning comprising nucleases, such as DNases. The compositions are preferably laundry detergent compositions and/or hard surface cleaning or treatment compositions, for example, dish-washing compositions, and are suitable for use in hand-washing or automatic washing. The invention also relates to methods of cleaning surfaces by hand and/or in automatic washing machines using such compositions, and a kit intended for cleaning or treatment, wherein the kit comprises a solution comprising a first DNase enzyme, a solution comprising a second DNase and optionally a cleaning or treatment adjunct.

BACKGROUND OF THE INVENTION

Most cleaning compositions (also known as detergents) for example, for hard surfaces and fabrics contain enzymes, typically a combination of various enzymes, each one targeting its specific substrate. Hard surfaces, fabric surfaces and even the interior of washing machines for cleaning such surfaces, in particular laundry washing machines and automatic dishwashing machines, are exposed to soils and microorganisms from the environment and from body soils such as soils comprising proteins, grease, starch and biofilm soils. Biofilm soils typically comprise a matrix of extracellular polymeric substance (EPS): a polymeric mixture generally comprising extracellular DNA, RNA, proteins, and polysaccharides. These soils are sticky, difficult to remove and tend to cause further adhesion of other soils. This is a particular problem for cleaning fabrics as such soils may tend to exacerbate redeposition of soils, resulting in fabric greying. Such soils may also cause malodour.

There is still a need for cleaning or treatment compositions which prevent, reduce or remove soil, in particular soil comprising biofilm, EPS, DNA and/or RNA. There is still a need for cleaning or treatment compositions which reduce malodour or a tendency for malodour to develop. There is still a need for cleaning or treatment compositions which reduce redeposition of soils (anti-redeposition). The present invention addresses one or more of these problems. The inventors have surprisingly found that a combination of at least two different DNases is particularly beneficial.

SUMMARY OF THE INVENTION

The present invention provides a cleaning or treatment composition comprising a first DNase, a second DNase and a cleaning or treatment adjunct. In one aspect the first DNase is an endonuclease or exonuclease and when the first DNase is an endonuclease the second DNase is an exonuclease, and when the first DNase is an exonuclease the second DNase is an endonuclease. In other words, the composition of the invention preferably comprises an endonuclease and an exonuclease.

The invention also provides a method of cleaning or otherwise treating a surface, preferably a fabric, the method comprising contacting the surface with an aqueous wash liquor comprising a first DNase, a second DNase and a cleaning or treatment adjunct; and then optionally rinsing the treated surface. Preferably the method is a method of cleaning.

The invention also provides use of a cleaning or treatment composition comprising a first DNase, a second DNase and a cleaning adjunct for cleaning of an item, wherein the item is a textile or a hard surface.

The invention also relates to a kit intended for cleaning or treatment, wherein the kit comprises a solution comprising a first DNase enzyme, a solution comprising a second DNase and optionally a cleaning or treatment adjunct, which may be in liquid form, for example in aqueous solution, or in dry form such as in the form of a powder/granule or other solid form. It may be preferred for the first DNase and the second DNase to be provided in the same aqueous solution, thus in a preferred aspect, the kit comprises an aqueous composition comprising a first DNase and a second DNase and a cleaning or treatment adjunct.

Preferably the surface is contacted with the aqueous wash liquor at a temperature of 60°C or less, or more preferably at a temperature of 40°C or less or 35°C or less, most preferably at a temperature of 30°C or less than 30°C, but greater than 5°C or greater than 10°C; and (iii) rinsing the surface. The compositions and methods herein are particularly useful for treating any surface, particularly fabrics and/or hard surfaces such as dishware, for example made from synthetic or natural materials, including cotton, wool, silk, polyester, nylon, elastane or mixed fabrics, such as polycotton.

The invention also relates to the use of a composition or method as described above for improved cleaning,

such as maintaining or improving whiteness of a fabric; improved soil removal from a surface, such as a fabric; malodour reduction or removal from a surface, such as a fabric; anti-wrinkle benefits on a fabric; improved drying of a fabric; soil anti-redeposition benefits, prevention and/or reduction of stickiness of a surface, pretreatment of stains, prevention and /or reduction of adherence of soil to a surface.

The invention also relates to a method of making a cleaning or treatment composition comprising preparing a polypeptide having DNase activity as described herein and mixing with a second DNase and a cleaning or treatment adjunct.

Preferably the first DNase and optionally, but preferably also the second DNase comprise one or more of the motif(s) selected from the group consisting of: [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [LV] [PTA] [FY] [DE] [VAGPH]D[CFY] [WY][AT][IM] L[CYQ] (SEQ ID NO 165), GPYCK (SEQ ID NO 166), WF[QE]IT (SEQ ID NO 167), [HQ][FILVY]X[GAQS]DX[HTGSA] [QVM]P[LFM]H (SEQ ID NO 168) and G[GA]NX[VILFY]X[VLM] (SEQ ID NO 169).

## DETAILED DESCRIPTION OF THE INVENTION

It will be apparent that the terms "first DNase" and "second DNase" are used herein to differentiate between the two different DNases in a given composition, and that a given DNase may in some cases, according to the context of the particular composition in which it is included, be considered as either a "first DNase" or a "second DNase". The term "two different DNases" means that the DNases do not share a sequence identity of 100%. Preferably the DNases have different cleavage patterns: e.g. endo and exo. The first DNase and the second DNase are preferably obtained from different genus, such that one is a bacterial DNase and the second is a fungal DNase. According to a further aspect, preferably the first DNase and second DNase are from different species, such as *Bacillus subtilis* and *Bacillus licheniformis.* Based on e.g. phylogenetic trees the DNases may be divided into various groups sharing structural and functional relationships. The nucleases for use in the current invention may be divided into the four groups NUC1_A, S1, NUC2_A and NUC2_A based on their structural and functional properties. Each group shares at least one common motif, which is a conserved sequence in the primary structure. According to a preferred aspect of the invention the first DNase and the second DNase are from a different sub-group selected from the four groups, NUC1_A, S1, NUC2_A and NUC2_A.

The inventors have found that combining two DNases e.g. from different subgroups, from different organisms and/or with different cleavage patterns results in improved cleaning performance e.g. stain removal of e.g. textiles. This effect may be termed boosting, as the improvement exceeds what could be achieved by increasing the dose of a single DNase. Thus, adding increasing amounts of a first DNase may not lead to improved stain removal (a performance plateau is reached) but adding a different DNase can improve the stain removal. There is an increased performance exceeding what could be achieved by simply overdosing the first DNase. In one embodiment, the DNases are from the same sub-group e.g. NUC1_A. In another embodiment, the DNases are from different sub-groups. The sub-groups may represent DNases having slightly different modes of action, e.g. cleavage specificity, substrate specificity etc.

The use of a second DNase from the same or different sub-group as defined in the current application boosts the performance of a first DNase. The invention thus relates to a composition comprising a first DNase and a second DNase. One preferred embodiment of the invention relates to a cleaning or treatment composition comprising a first DNase, a second DNase and a cleaning or treatment adjunct.

The DNases of the invention preferably have different modes of action, examples of which are different cleavage patterns, such as endo or exo-cleavage of the DNA substrate, DNases of different origin, e.g. from bacteria or fungi, or DNases from different subgroups e.g. NUC1 or NUC2, wherein each group shares at least one conserved structural motif.

One embodiment of the invention relates to a composition comprising a first DNase and a second DNase, wherein the DNases are endonucleases.

One embodiment of the invention relates to a composition comprising a first DNase and a second DNase, wherein the DNases are exonucleases.

In a preferred embodiment, the first and the second DNase have different cleavage patterns, e.g. not both endo- or not both exonucleases.

One embodiment of the invention relates to a composition comprising a first DNase and a second DNase, wherein the first DNase is an endonuclease or exonuclease and wherein, when the first DNase is an endonuclease the second DNase is an exonuclease, and when the first DNase is an exonuclease the second DNase is an endonuclease. In other words, the composition of this embodiment comprises at least one endonuclease and at least one exonuclease.

One preferred embodiment of the invention relates to a cleaning or treatment composition comprising a first DNase, a second DNase and a cleaning or treatment adjunct, wherein the DNases are endonucleases.

One preferred embodiment of the invention relates to a cleaning or treatment composition comprising a first DNase, a second DNase and a cleaning or treatment adjunct, wherein the DNases are exonucleases.

One preferred embodiment of the invention relates to a cleaning or treatment composition comprising a first

DNase, a second DNase and a cleaning or treatment adjunct, wherein the first DNase is an endonuclease or exonuclease and wherein, when the first DNase is an endonuclease the second DNase is an exonuclease, and when the first DNase is an exonuclease the second DNase is an endonuclease. In other words, the composition of this embodiment comprises at least one endonuclease and at least one exonuclease.

The DNase to be combined in a composition of the invention may also be derived from different sources. DNases of different origin and polypeptide composition can have different specificities in degrading DNA, such as endo- and eco-acting, and will complement each other in removal of DNA.

Preferably according to the invention comprising a first DNase and a second DNase, at least one DNase is bacterial.

One embodiment of the invention relates to a composition comprising a first DNase and a second DNase, wherein at least one DNase is obtained from bacteria, preferably *Bacillus* e.g. *Bacillus licheniformis, Bacillus amylolichenifaciens, Bacillus algicola, Bacillus vietnamensis, vhwajinpoensis, Bacillus halodurans, Bacillus idriensis, Bacillus indicus, Bacillus luciferensis, Bacillus cibi, Bacillus marisflavi, Bacillus compisalis or Bacillus horikoshii.*

One embodiment of the invention relates to a composition comprising a first DNase and a second DNase, wherein the first and the second DNase are obtained from bacteria preferably of the genus *Bacillus,* e.g. *Bacillus licheniformis, Bacillus amylolichenifaciens, Bacillus algicola, Bacillus vietnamensis, vhwajinpoensis, Bacillus halodurans, Bacillus idriensis, Bacillus indicus, Bacillus luciferensis, Bacillus cibi, Bacillus marisflavi, Bacillus compisalis or Bacillus horikoshii.*

Preferably according to the invention the composition comprises a first DNase and a second DNase, wherein at least one DNase is fungal.

One embodiment of the invention relates to a composition comprising a first DNase and a second DNase, wherein at least one DNase is obtained from a fungus, preferably obtained from *Aspergillus, Rhizoctonia, Trichoderma* or *Morchella,* e.g. *Aspergillus oryzae, Rhizoctonia solani, Trichoderma harzianum, Morchella crassipes, Morchella esculenta* or *Morchella costata.*

One embodiment of the invention relates to a composition comprising a first DNase and a second DNase, wherein the first and the second DNase are fungal, preferably obtained from *Aspergillus, Rhizoctonia, Trichoderma* or *Morchella,* e.g. *Aspergillus oryzae, Rhizoctonia solani, Trichoderma harzianum, Morchella crassipes, Morchella esculenta* or *Morchella costata.*

One embodiment of the invention relates to a composition comprising a first DNase and a second DNase, wherein at least one DNase is bacterial and wherein at least one DNase is fungal.

One embodiment of the invention relates to a composition comprising a first DNase and a second DNase, wherein at least one DNase is obtained from bacteria, preferably *Bacillus,* e.g. *Bacillus licheniformis, Bacillus amylolichenifaciens, Bacillus algicola, Bacillus vietnamensis, vhwajinpoensis, Bacillus halodurans, Bacillus idriensis, Bacillus indicus, Bacillus luciferensis, Bacillus cibi, Bacillus marisflavi, Bacillus compisalis or Bacillus horikoshii,* and wherein at least one DNase is fungal, preferably obtained from *Aspergillus, Rhizoctonia, Trichoderma* or *Morchella,* e.g. *Aspergillus oryzae, Rhizoctonia solani, Trichoderma harzianum, Morchella crassipes, Morchella esculenta* or *Morchella costata.*

The compositions of the invention are cleaning or treatment compositions comprising at least one cleaning or treatment adjunct.

The DNases to be included in a composition of the invention may belong to different subgroups, also termed clusters. Enzymes may be divided into sub-groups depending on their structural and functional properties. The polypeptides comprising deoxyribonuclease activity of the current invention may be divided in the sub-groups listed below.

NUC1_A nuclease

The NUC1_A nucleases comprise the domain DUF1524, as defined in PFAM (PF07510, Pfam version 30.0 Finn (2016). Nucleic Acids Research, Database Issue 44:D279-D285). The polypeptides comprising the DUF1524 domain can be separated into distinct sub-clusters, where we previously denoted one sub-cluster comprising the motif [F/L/Y/I]A[N/R]D[L/I/P/V][ (SEQ ID NO 158), located at positions corresponding to positions 111 to 115 of SEQ ID NO 1 as family NUC1, see further in WO2017/060475 (Novozymes A/S). Another motif characteristic of this sub-cluster is C[D/N]T[A/R] (SEQ ID NO 159), located at positions corresponding to positions 44 to 47 of (SEQ ID NO 1). In addition to comprising any of the motifs above, the polypeptides having DNase activity belonging to the NUC1_A domain share the common motif [D/Q][I/V]DH (SEQ ID NO 160), corresponding to amino acid 85 to 88 in the reference polypeptide (SEQ ID NO: 1). The nucleases shown in SEQ ID Nos: 1 to 102 are defined as NUC1_A DNases.

NUC2_A and NUC2 B

The nuclease domain comprised in the polypeptides SEQ ID Nos: 103 to 141 of the invention may be classified as exo_endo_phos nucleases, containing an exo_endo_phos domain (Pfam domain id PF03372, Pfam version 31.0

Finn (2016). Nucleic Acids Research, Database Issue 44: D279-D285). The exo_endo_phos nucleases are a structural superfamily containing nucleases with endonuclease, exonuclease and phosphatase activities. The nucleases share a common catalytic mechanism of cleaving phosphodiester bonds. The NUC2 nucleases are exo_endo_phos nucleases comprising the motif [G/Y/W/F/A/H]NI[R/Q/D/E/V] (SEQ ID NO 161). The nucleotides in NUC2 can be separated into at least two distinct sub-clusters, based on structural and functional similarities, which are denoted NUC2_A and NUC2_B.

The subgroup termed NUC2_A comprises the polypeptides shown in SEQ ID 103-107. The NUC2_A nucleases are annotated as Conserved Protein Domain Family EEP-1 (domain ID cd09083, Marchler-Bauer, CDD/SPARCLE: functional classification of proteins via subfamily domain architectures, Nucleic Acids Res. 45:D200-D203, 2017). The Nucleases of NUC2_A comprise the motif GR[DN][DN]G (SEQ ID NO: 162) corresponding to positions 100 to 104 in *Cadophora fastigiate* (SEQ ID NO 103).

The sub-group NUC2_B is defined with motif SDH[D/H/L]P (SEQ ID NO 163), corresponding to position 577 to 581 of SEQ ID NO: 141. The polypeptides of the NUC2_B domain may also comprise the motif GGNI[R/Q] (SEQ ID NO 164), corresponding to positions 368 to 372 in *Sporormia fimetaria* (SEQ ID NO: 141). Nucleases belonging to the NUC2_B domain group share the above motifs, which are thus common to the DNase polypeptides in the NUC2_B cluster. Examples of DNases of the invention of the NUC2_B domain group are DNases comprising the polypeptides shown in SEQ ID NO 108 to SEQ ID NO 141.

NUC3

The nuclease domain comprised in the polypeptides of SEQ ID Nos 151 to 157 of the invention may be classified as NUC3 nucleases.

The NUC3 nucleases may comprise the motif [LV] [PTA] [FY] [DE] [VAGPH]D[CFY] [WY] [AT][IM]L[CYQ] (SEQ ID NO 165) corresponding to positions 24 to 35 in *Aspergillus oryzae* (SEQ ID NO 151), and/or any of the motifs GPYCK (SEQ ID NO 166) corresponding to positions 157 to 161 in *Aspergillus oryzae* (SEQ ID NO 151) or WF[QE]IT (SEQ ID NO 167) corresponding to positions 146 to 150 in *Aspergillus oryzae* (SEQ ID NO 151)

S1P1_nucleases

The nuclease domain comprised in the polypeptides SEQ ID 142 to 150 of the invention may be classified as S1-P1 nucleases (Pfam domain id PF02265, Pfam version 31.0 Finn (2016). Nucleic Acids Research, Database Issue 44:D279-D285). The S1-P1 nuclease domain is a functional domain providing hydrolytic activity to the polypeptide. The S1-P1 nucleases cleave single stranded DNA and RNA with no base specificity and may also introduce single-stranded breaks in double-stranded DNA or RNA, or DNA-RNA hybrids. EC:3.1.30.1: "*Aspergillus nuclease* S(1)".

Polypeptides of S1P1 comprise one or both the motifs [HQ][FILVY]X[GAQS]DX[HTGSA][QVM]P[LFM]H (SEQ ID NO 168), situated in positions corresponding to positions 116 to 126 in *Trichoderma hamatum* (SEQ ID NO 150) and/or G[GA]NX[VILFY]X[VLM] (SEQ ID NO 169), located at positions 133 to 139 in SEQ ID NO 150.

The sequences SEQ ID NO 1 to SEQ ID NO 102 are DNases belonging to the NUC1_A group of DNases, which are preferably combined with any of the NUC2_A, NUC2_B or S1 DNases. The sequences shown in SEQ ID NO 103 to SEQ ID NO 107 are DNases belonging to NUC2_A group of DNases, sequences shown in SEQ ID NO 108 to SEQ ID NO 141 are DNases belonging to the NUC2_B group of DNases, sequences shown in SEQ ID NO 142 to SEQ ID NO 150 belong to the S1P1 group of DNases, and the sequence shown in SEQ ID NO 151 belongs to the NUC3 group, as shown in Tables 1, 2, 3, 4 and 5 below.

Table 1. NUC1_A DNases

| SEQ ID NO | Organism |
|---|---|
| 1 | *Bacillus cibi* |
| 2 | *Rhizoctonia solani* |
| 3 | *Disciotis venosa* |
| 4 | *Gyromitra esculenta* |
| 5 | *Phlebia subochracea* |
| 6 | *Rhizoctonia solani* |
| 7 | *Rhizoctonia solani* |
| 8 | *Morchella crassipes* |

(continued)

| SEQ ID NO | Organism |
|---|---|
| 9 | *Morchella esculenta* |
| 10 | *Rhizoctonia solani* |
| 11 | *Streptomyces sp-63712* |
| 12 | *Irpex lacteus* |
| 13 | *Stropharia semiglobata* |
| 14 | *Cladosporium cladosporioides* |
| 15 | *Stropharia semiglobata* |
| 16 | *Physisporinus sanguinolentus* |
| 17 | *Streptomyces laculatispora* |
| 18 | *Zopfiella sp. G367* |
| 19 | *Didymosphaeria futilis* |
| 20 | *Phaeosphaeria sp.* |
| 21 | *Pleosporales* |
| 22 | *Bacillus horikoshii* |
| 23 | *Metarhizium lepidiotae* |
| 24 | *Mortierella humilis* |
| 25 | *Vibrissea flavovirens* |
| 26 | *Alternaria sp.* |
| 27 | *Streptomyces thermoalcalitolerans* |
| 28 | *Deconica coprophila* |
| 29 | *Bacillus campisalis* |
| 30 | *Bacillus marisflavi* |
| 31 | *Bacillus horikoshii* |
| 32 | *Phialophora geniculata* |
| 33 | *Aspergillus lentulus* |
| 34 | *Penicillium sp-44220* |
| 35 | *Alternaria sp. XZ2545* |
| 36 | *Scytalidium circinatum* |
| 37 | *Bacillus luciferensis* |
| 38 | *Bacillus sp-62520* |
| 39 | *Roussoella intermedia* |
| 40 | *Cercospora fusimaculans* |
| 41 | *Penicillium cremeogriseum* |
| 42 | *Thermoactinomyces daqus* |
| 43 | *Penicillium reticulisporum* |
| 44 | *Urnula sp-56769* |
| 45 | *Bacillus marisflavi* |
| 46 | *Cordyceps tenuipes* |

(continued)

| SEQ ID NO | Organism |
|---|---|
| 47 | *Bacillus indicus* |
| 48 | *Arthrographis sp. 07MA20* |
| 49 | *Neosartorya massa* |
| 50 | *Trichophaea abundans* |
| 51 | *Ascobolus sp. ZY179* |
| 52 | *Acremonium sp. XZ2414* |
| 53 | *Trichophaea minuta* |
| 54 | *Trichophaea minuta* |
| 55 | *Rhizoctonia solani* |
| 56 | *Monilinia fructicola* |
| 57 | *Corynespora cassiicola* |
| 58 | *Humicolopsis cephalosporioides* |
| 59 | *Halobacillus karajensis* |
| 60 | *Endophragmiella valdina* |
| 61 | *Bacillus sp. EB01* |
| 62 | *Metarhizium sp. HNA15-2* |
| 63 | *Morchella costata* |
| 64 | *Sarocladium sp. XZ2014* |
| 65 | *Bacillus sp-62668* |
| 66 | *Marasmius oreades* |
| 67 | *Acremonium dichromosporum* |
| 68 | Enviromental sample O |
| 69 | *Bipolaris zeicola* |
| 70 | *Acremonium sp. XZ1968* |
| 71 | *Acremonium sp. XZ2007* |
| 72 | *Pholiota squarrosa* |
| 73 | *Actinomadura rifamycini* |
| 74 | Enviromental sample D |
| 75 | *Vibrissea flavovirens* |
| 76 | *Rhizoctonia solani* |
| 77 | *Pyrenochaetopsis sp.* |
| 78 | *Bacillus sp-62451* |
| 79 | *Trichobolus zukalii* |
| 80 | *Bacillus indicus* |
| 81 | *Daldinia fissa* |
| 82 | *Metapochonia suchlasporia* |
| 83 | *Trichophaea saccata* |
| 84 | *Bacillus algicola* |

(continued)

| SEQ ID NO | Organism |
|---|---|
| 85 | *Pycnidiophora cf.dispera* |
| 86 | *Mycothermus thermophilus* |
| 87 | *Bacillus hwajinpoensis* |
| 88 | *Saccharothrix australiensis* |
| 89 | *Paenibacillus mucilaginosus* |
| 90 | *Ascobolus stictoideus* |
| 91 | *Trichoderma hamatum* |
| 92 | *Pseudoplectania nigrella* |
| 93 | *Chaetomium thermophilum var. thermophilum* |
| 94 | *Bacillus vietnamensis* |
| 95 | *Bacillus sp-11238* |
| 96 | *Glycomyces rutgersensis* |
| 97 | *Bacillus horikoshii* |
| 98 | *Kutzneria albida* |
| 99 | *Bacillus idriensis* |
| 100 | *Sporormia fimetaria* |
| 101 | *Thermobispora bispora* |
| 102 | *Trichoderma reesei* |

Table 2. NUC2_A DNases

| SEQ ID NOs | Organism |
|---|---|
| 103 | *Cadophora fastigiata* |
| 104 | *Phialophora geniculata* |
| 105 | *Stenocarpella maydis* |
| 106 | *Pyrenochaetopsis sp.* |
| 107 | *Pseudoplectania vogesiaca* |

Table 3. NUC2_B DNases

| SEQ ID NOs | Organism |
|---|---|
| 108 | *Achaetomium luteum* |
| 109 | *Mycothermus thermophilus* |
| 110 | *Stenocarpella maydis* |
| 111 | *Chaetomium ancistrocladum* |
| 112 | *Aspergillus insuetus* |
| 113 | *Stachybotrys sp. 12501* |
| 114 | *Arthrinium arundinis* |
| 115 | *Phialophora geniculata* |

(continued)

| SEQ ID NOs | Organism |
|---|---|
| 116 | *Aspergillus iizukae* |
| 117 | *Aspergillus niger* |
| 118 | *Plectosphaerella sp. 1-29* |
| 119 | *Lecanicillium psalliotae* |
| 120 | *Simplicillium obclavatum* |
| 121 | *Hypoxylon sp.* |
| 122 | *Aspergillus aculeatus* |
| 123 | *Warcupiella spinulosa* |
| 124 | *Acremonium sp. XZ2020* |
| 125 | *Preussia aemulans* |
| 126 | *Exserohilum rostratum* |
| 127 | *Trichurus spiralis* |
| 128 | *Acremonium sp. XZ1982* |
| 129 | *Physalacria cryptomeriae* |
| 130 | *Aspergillus sydowii* |
| 131 | *Corynascus sepedonium* |
| 132 | *Colletotrichum circinans* |
| 133 | *Paradendryphiella salina* |
| 134 | *Daldinia fissa* |
| 135 | *Purpureocillium lilacinum* |
| 136 | *Fusarium neocosmosporiellum* |
| 137 | *Acrophialophora fusispora* |
| 138 | *Pyronema domesticum* |
| 139 | *Rhinocladiella sp.* |
| 140 | *Trametes cinnabarina* |
| 141 | *Sporormia fimetaria* |

Table 4. S1P1 DNases

| SEQ ID NOs | Organism |
|---|---|
| 142 | *Cadophora fastigiata* |
| 143 | *Phialophora geniculata* |
| 144 | *Cordyceps cardinalis* |
| 145 | *Stenocarpella maydis* |
| 146 | *Stenocarpella maydis* |
| 147 | *Penicillium cremeogriseum* |
| 148 | *Trichoderma reesei* |
| 149 | *Morchella costata* |

(continued)

| SEQ ID NOs | Organism |
|---|---|
| 150 | *Trichoderma hamatum* |

Table 5. NUC 3 DNases

| SEQ ID Nos | Organism |
|---|---|
| 151 | *Aspergillus oryzae* |
| 152 | *Aspergillus chevalieri* |
| 153 | *Aspergillus tubingensis* |
| 154 | *Aspergillus parasiticus* |
| 155 | *Metarhizium anisopliae* |
| 156 | *Cordyceps fumosorosea* |
| 157 | *Aspergillus oryzae* |

A DNase from Table 1 is preferably combined with any of the DNases of Tables 2, 3, 4 and 5. In one preferred embodiment, any of the DNases shown in Table 1 is combined with any of the DNases shown in Table 2. In one preferred embodiment, any of the DNases shown in Table 1 is combined with any of the DNases shown in Table 3. In one preferred embodiment, any of the DNases shown in Table 1 is combined with any of the DNases shown in Table 4. In one preferred embodiment, any of the DNases shown in Table 1 is combined with any of the DNases shown in Table 5.

In one embodiment, a NUC1_A DNase, preferably selected from the group of DNases consisting of DNases shown in Table 1, is combined with a NUC2_A DNase selected from the group consisting of DNases shown in Table 2. In one embodiment, a NUC1_A DNase, preferably selected from the group of DNases consisting of DNases shown in Table 1, is combined with a NUC2_B DNase selected from the group consisting of DNases shown in Table 3. In one embodiment, a NUC1_A DNase, preferably selected from the group of DNases consisting of DNases shown in Table 1, is combined with an S1P1 DNase selected from the group consisting of DNases shown in Table 4. In one embodiment, a NUC1 _A DNase, preferably selected from the group of DNases consisting of DNases shown in Table 1, is combined with a NUC 3 DNase selected from the group consisting of DNases shown in Table 5.

The DNases of Table 2 are preferably combined with any of the DNases of Tables 3 and 4. In one preferred embodiment, any of the DNases shown in Table 2 is combined with any of the DNases shown in Table 3. In one preferred embodiment, any of the DNases shown in Table 2 is combined with any of the DNases shown in Table 4.

In one embodiment, a NUC2_A DNase, preferably selected from the group of DNases consisting of DNases shown in Table 2, is combined with a NUC2_B DNase selected from the group consisting of DNases shown in Table 3. In one embodiment, a NUC2_A DNase, preferably selected from the group of DNases consisting of DNases shown in Table 2, is combined with an S1P1 DNase selected from the group consisting of DNases shown in Table 4. In one embodiment, a NUC2_A DNase, preferably selected from the group of DNases consisting of DNases shown in Table 2, is combined with a NUC 3 DNase selected from the group consisting of DNases shown in Table 5.

The DNases of Table 3 are preferably combined with any of the DNases of Table 4 or Table 5. In one preferred embodiment, any of the DNases shown in Table 3 is combined with any of the DNases shown in Table 4. In one preferred embodiment, any of the DNases shown in Table 3 is combined with any of the DNases shown in Table 5.

In one embodiment, a NUC2_B DNase, preferably selected from the group of DNases consisting of DNases shown in Table 3, is combined with an S1P1 DNase selected from the group consisting of DNases shown in Table 4. In one embodiment, a NUC2_B DNase, preferably selected from the group of DNases consisting of DNases shown in Table 3, is combined with a NUC 3 DNase selected from the group consisting of DNases shown in Table 5.

The DNases of Table 4 are preferably combined with any of the DNases of Table 5. In one preferred embodiment, any of the DNases shown in Table 4 is combined with any of the DNases shown in Table 5

In one embodiment, an S1P1 DNase, preferably selected from the group of DNases consisting of DNases shown in Table 4, is combined with a NUC 3 DNase selected from the group consisting of DNases shown in Table 5.

One embodiment of the invention relates to a composition comprising a first DNase and a second DNase, wherein at least one DNase is a NUC1_A, a NUC2_A, NUC2_B, NUC3 or S1P1 DNase.

One embodiment of the invention relates to a composition comprising a first DNase and a second DNase, wherein the first DNase is a NUC1_A DNase and the second DNase is a NUC2_A DNase

One embodiment of the invention relates to a composition comprising a first DNase and a second DNase, wherein the first DNase is a NUC1_A DNase and the second DNase is a NUC2_B DNase.

One embodiment of the invention relates to a composition comprising a first DNase and a second DNase, wherein the first DNase is a NUC1_A DNase and the second DNase is a NUC3 DNase.

One embodiment of the invention relates to a composition comprising a first DNase and a second DNase, wherein the first DNase is a NUC1_A DNase and the second DNase is an S1P1 DNase.

One embodiment of the invention relates to a composition comprising a first DNase and a second DNase, wherein the first DNase is a NUC2_A DNase and the second DNase is a NUC2_B DNase.

One embodiment of the invention relates to a composition comprising a first DNase and a second DNase, wherein the first DNase is a NUC2_A DNase and the second DNase is a NUC3 DNase.

One embodiment of the invention relates to a composition comprising a first DNase and a second DNase, wherein the first DNase is a NUC2_A DNase and the second DNase is an S1P1 DNase.

One embodiment of the invention relates to a composition comprising a first DNase and a second DNase, wherein the first DNase is a NUC2_B DNase and the second DNase is a NUC3 DNase.

One embodiment of the invention relates to a composition comprising a first DNase and a second DNase, wherein the first DNase is a NUC2_B DNase and the second DNase is an S1P1 DNase.

One embodiment of the invention relates to a composition comprising a first DNase and a second DNase, wherein the first DNase is a NUC3 DNase and the second DNase is an S1P1 DNase.

One embodiment of the invention relates to a cleaning or treatment composition comprising a first DNase, a second DNase and at least one cleaning or treatment adjunct, wherein at least one DNase is a NUC1_A, a NUC2_A, NUC2_B, NUC3 or S1P1 DNase.

One embodiment of the invention relates to a cleaning or treatment composition comprising a first DNase, a second DNase and at least one cleaning or treatment adjunct, wherein the first DNase is a NUC1_A DNase and the second DNase is a NUC2_A DNase

One embodiment of the invention relates to a cleaning or treatment composition comprising a first DNase, a second DNase and at least one cleaning or treatment adjunct, wherein the first DNase is a NUC1_A DNase and the second DNase is a NUC2_B DNase.

One embodiment of the invention relates to a cleaning or treatment composition comprising a first DNase, a second DNase and at least one cleaning or treatment adjunct, wherein the first DNase is a NUC1_A DNase and the second DNase is a NUC3 DNase.

One embodiment of the invention relates to a cleaning or treatment composition comprising a first DNase, a second DNase and at least one cleaning or treatment adjunct, wherein the first DNase is a NUC1_A DNase and the second DNase is an S1P1 DNase.

One embodiment of the invention relates to a cleaning or treatment composition comprising a first DNase, a second DNase and at least one cleaning or treatment adjunct, wherein the first DNase is a NUC2_A DNase and the second DNase is a NUC2_B DNase.

One embodiment of the invention relates to a cleaning or treatment composition comprising a first DNase, a second DNase and at least one cleaning or treatment adjunct, wherein the first DNase is a NUC2_A DNase and the second DNase is a NUC3 DNase.

One embodiment of the invention relates to a cleaning or treatment composition comprising a first DNase, a second DNase and at least one cleaning or treatment adjunct, wherein the first DNase is a NUC2_A DNase and the second DNase is an S1P1 DNase.

One embodiment of the invention relates to a cleaning or treatment composition comprising a first DNase, a second DNase and at least one cleaning or treatment adjunct, wherein the first DNase is a NUC2_B DNase and the second DNase is a NUC3 DNase.

One embodiment of the invention relates to a cleaning or treatment composition comprising a first DNase, a second DNase and at least one cleaning or treatment adjunct, wherein the first DNase is a NUC2_B DNase and the second DNase is an S1P1 DNase.

One embodiment of the invention relates to a cleaning or treatment composition comprising a first DNase, a second DNase and at least one cleaning or treatment adjunct, wherein the first DNase is a NUC3 DNase and the second DNase is an S1P1 DNase.

One embodiment of the invention relates to a composition comprising a first DNase and a second DNase, wherein the first and the second DNase comprise one or more of the motif(s) selected from the group consisting of: [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [LV] [PTA] [FY] [DE] [VAGPH]D[CFY] [WY][AT][IM]L[CYQ] (SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

One embodiment of the invention relates to a composition, preferably a cleaning composition, comprising a first DNase and a second DNase, wherein the first DNase comprises one or more motif(s) selected from the group consisting of: [F/L/Y/I]A[N/R]D[L/I/P/V][ (SEQ ID NO 158), C[D/N]T[A/R] (SEQ ID NO 159) and [D/Q][I/V]DH (SEQ ID NO 160), and wherein the second DNase comprises one or both motif(s) [G/Y/W/F/A/H]NI[R/Q/D/E/V] (SEQ ID NO 161) and/or GR[DN][DN]G (SEQ ID NO: 162).

One embodiment of the invention relates to a composition, preferably a cleaning composition, comprising a first DNase and a second DNase, wherein the first DNase comprises one or more motif(s) selected from the group consisting of: [F/L/Y/I]A[N/R]D[L/I/P/V][ (SEQ ID NO 158), C[D/N]T[A/R] (SEQ ID NO 159) and [D/Q][I/V]DH (SEQ ID NO 160), and wherein the second DNase comprises one or both motif(s) SDH[D/H/L]P (SEQ ID NO 163) and/or GGNI[R/Q] (SEQ ID NO 164).

One embodiment of the invention relates to a composition, preferably a cleaning composition, comprising a first DNase and a second DNase, wherein the first DNase comprises one or more motif(s) selected from the group consisting of: [F/L/Y/I]A[N/R]D[L/I/P/V][ (SEQ ID NO 158), C[D/N]T[A/R] (SEQ ID NO 159) and [D/Q][I/V]DH (SEQ ID NO 160), and wherein the second DNase comprises one or more motif(s) selected from the group consisting of: [LV] [PTA][FY] [DE][VAGPH]D[CFY] [WY] [AT] [IM]L[CYQ] (SEQ ID NO 165), GPYCK (SEQ ID NO 166) and WF[QE]IT (SEQ ID NO 167).

One embodiment of the invention relates to a composition, preferably a cleaning composition, comprising a first DNase and a second DNase, wherein the first DNase comprises one or more motif(s) selected from the group consisting of: [F/L/Y/I]A[N/R]D[L/I/P/V][ (SEQ ID NO 158), C[D/N]T[A/R] (SEQ ID NO 159) and [D/Q][I/V]DH (SEQ ID NO 160), and wherein the second DNase comprises one or more motif(s) selected from the group consisting of: [HQ] [FILVY]X[GAQS]DX[HTGSA][QVM]P[LFM]H (SEQ ID NO 168) and G[GA]NX[VILFY]X[VLM] (SEQ ID NO 168).

One embodiment of the invention relates to a composition, preferably a cleaning composition, comprising a first DNase and a second DNase, wherein the first DNase comprises one or both motif(s) [G/Y/W/F/A/H]NI[R/Q/D/E/V] (SEQ ID NO 161) and/or GR[DN][DN]G (SEQ ID NO: 162) and wherein the second DNase comprises one or both motif(s) SDH[D/H/L]P (SEQ ID NO 163) and/or GGNI[R/Q] (SEQ ID NO 164).

One embodiment of the invention relates to a composition, preferably a cleaning composition, comprising a first DNase and a second DNase, wherein the first DNase comprises one or both motif(s) [G/Y/W/F/A/H]NI[R/Q/D/E/V] (SEQ ID NO 161) and/or GR[DN][DN]G (SEQ ID NO: 162) and wherein the second DNase comprises one or more motif(s) selected from the group consisting of: [LV][PTA][FY][DE][VAGPH]D[CFY][WY][AT][IM]L[CYQ] (SEQ ID NO 165), GPYCK (SEQ ID NO 166) and WF[QE]IT (SEQ ID NO 167).

One embodiment of the invention relates to a composition, preferably a cleaning composition, comprising a first DNase and a second DNase, wherein the first DNase comprises one or both motif(s) [G/Y/W/F/A/H]NI[R/Q/D/E/V] (SEQ ID NO 161) and/or GR[DN][DN]G (SEQ ID NO: 162) and wherein the second DNase comprises one or more motif(s) selected from the group consisting of: [HQ][FILVY]X[GAQS]DX[HTGSA][QVM]P[LFM]H (SEQ ID NO 168) and G[GA]NX[VILFY]X[VLM] (SEQ ID NO 169).

One embodiment of the invention relates to a composition, preferably a cleaning composition, comprising a first DNase and a second DNase, wherein the first DNase comprises one or both motif(s) SDH[D/H/L]P (SEQ ID NO 163) and/or GGNI[R/Q] (SEQ ID NO 164) and wherein the second DNase comprises one or more motif(s) selected from the group consisting of: [LV] [PTA] [FY] [DE] [VAGPH]D[CFY] [WY][AT][IM]L[CYQ] (SEQ ID NO 165), GPYCK (SEQ ID NO 166) and WF[QE]IT (SEQ ID NO 167).

One embodiment of the invention relates to a composition, preferably a cleaning composition, comprising a first DNase and a second DNase, wherein the first DNase comprises one or both motif(s) SDH[D/H/L]P (SEQ ID NO 163) and/or GGNI[R/Q] (SEQ ID NO 164) and wherein the second DNase comprises one or more motif(s) selected from the group consisting of: [HQ][FILVY]X[GAQS]DX[HTGSA][QVM]P[LFM]H (SEQ ID NO 168) and G[GA]NX[VILFY]X[VLM] (SEQ ID NO 169).

One embodiment of the invention relates to a composition, preferably a cleaning composition, comprising a first DNase and a second DNase, wherein the first DNase comprises one or more motif(s) selected from the group consisting of: [LV] [PTA] [FY] [DE] [VAGPH]D[CFY] [WY][AT][IM]L[CYQ] (SEQ ID NO 165), GPYCK (SEQ ID NO 166) and WF[QE]IT (SEQ ID NO 167) and wherein the second DNase comprises one or more motif(s) selected from the group consisting of: [HQ][FILVY]X[GAQS]DX[HTGSA][QVM]P[LFM]H (SEQ ID NO 168) and G[GA]NX[VILFY]X[VLM] (SEQ ID NO 169).

In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with another NUC1_A DNase. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with another NUC1_A DNase, wherein the DNase is selected from the group consisting of DNases shown in Table 1.

In one embodiment, a DNase having at least 60%, at least 65%, at least 70%, at least 75%, at least 80%, at

least 85%, at least 90%, at least 95%, at least 98% or 100% sequence identity to the amino acid sequence shown in SEQ ID NO 1 is combined with a DNase selected from the group consisting of:

a) a DNase having at least 60%, at least 65%, at least 70%, at least 75%, at least 80%, at least 85%, at least 90%, at least 95%, at least 98% or 100% sequence identity to the amino acid sequence shown in SEQ ID NO 2,
b) a DNase having at least 60%, at least 65%, at least 70%, at least 75%, at least 80%, at least 85%, at least 90%, at least 95%, at least 98% or 100% sequence identity to the amino acid sequence shown in SEQ ID NO 3,
c) a DNase having at least 60%, at least 65%, at least 70%, at least 75%, at least 80%, at least 85%, at least 90%, at least 95%, at least 98% or 100% sequence identity to the amino acid sequence shown in SEQ ID NO 4,
d) a DNase having at least 60%, at least 65%, at least 70%, at least 75%, at least 80%, at least 85%, at least 90%, at least 95%, at least 98% or 100% sequence identity to the amino acid sequence shown in SEQ ID NO 5,
e) a DNase having at least 60%, at least 65%, at least 70%, at least 75%, at least 80%, at least 85%, at least 90%, at least 95%, at least 98% or 100% sequence identity to the amino acid sequence shown in SEQ ID NO 6,
f) a DNase having at least 60%, at least 65%, at least 70%, at least 75%, at least 80%, at least 85%, at least 90%, at least 95%, at least 98% or 100% sequence identity to the amino acid sequence shown in SEQ ID NO 7,
g) a DNase having at least 60%, at least 65%, at least 70%, at least 75%, at least 80%, at least 85%, at least 90%, at least 95%, at least 98% or 100% sequence identity to the amino acid sequence shown in SEQ ID NO 8,
h) v a DNase having at least 60%, at least 65%, at least 70%, at least 75%, at least 80%, at least 85%, at least 90%, at least 95%, at least 98% or 100% sequence identity to the amino acid sequence shown in SEQ ID NO 9,
i) a DNase having at least 60%, at least 65%, at least 70%, at least 75%, at least 80%, at least 85%, at least 90%, at least 95%, at least 98% or 100% sequence identity to the amino acid sequence shown in SEQ ID NO 10,
j) v a DNase having at least 60%, at least 65%, at least 70%, at least 75%, at least 80%, at least 85%, at least 90%, at least 95%, at least 98% or 100% sequence identity to the amino acid sequence shown in SEQ ID NO 11,
k) a DNase having at least 60%, at least 65%, at least 70%, at least 75%, at least 80%, at least 85%, at least 90%, at least 95%, at least 98% or 100% sequence identity to the amino acid sequence shown in SEQ ID NO 12,
l) a DNase having at least 60%, at least 65%, at least 70%, at least 75%, at least 80%, at least 85%, at least 90%, at least 95%, at least 98% or 100% sequence identity to the amino acid sequence shown in SEQ ID NO 13,
m) a DNase having at least 60%, at least 65%, at least 70%, at least 75%, at least 80%, at least 85%, at least 90%, at least 95%, at least 98% or 100% sequence identity to the amino acid sequence shown in SEQ ID NO 14,
n) a DNase having at least 60%, at least 65%, at least 70%, at least 75%, at least 80%, at least 85%, at least 90%, at least 95%, at least 98% or 100% sequence identity to the amino acid sequence shown in SEQ ID NO 15,
o) a DNase having at least 60%, at least 65%, at least 70%, at least 75%, at least 80%, at least 85%, at least 90%, at least 95%, at least 98% or 100% sequence identity to the amino acid sequence shown in SEQ ID NO 16,
p) a DNase having at least 60%, at least 65%, at least 70%, at least 75%, at least 80%, at least 85%, at least 90%, at least 95%, at least 98% or 100% sequence identity to the amino acid sequence shown in SEQ ID NO 17,
q) a DNase having at least 60%, at least 65%, at least 70%, at least 75%, at least 80%, at least 85%, at least 90%, at least 95%, at least 98% or 100% sequence identity to the amino acid sequence shown in SEQ ID NO 18,
r) a DNase having at least 60%, at least 65%, at least 70%, at least 75%, at least 80%, at least 85%, at least 90%, at least 95%, at least 98% or 100% sequence identity to the amino acid sequence shown in SEQ ID NO 19,
s) a DNase having at least 60%, at least 65%, at least 70%, at least 75%, at least 80%, at least 85%, at least 90%, at least 95%, at least 98% or 100% sequence identity to the amino acid sequence shown in SEQ ID NO 20,
t) a DNase having at least 60%, at least 65%, at least 70%, at least 75%, at least 80%, at least 85%, at least 90%, at least 95%, at least 98% or 100% sequence identity to the amino acid sequence shown in SEQ ID NO 21,
u) a DNase having at least 60%, at least 65%, at least 70%, at least 75%, at least 80%, at least 85%, at least 90%, at least 95%, at least 98% or 100% sequence identity to the amino acid sequence shown in SEQ ID NO 22,
v) a DNase having at least 60%, at least 65%, at least 70%, at least 75%, at least 80%, at least 85%, at least 90%, at least 95%, at least 98% or 100% sequence identity to the amino acid sequence shown in SEQ ID NO 23,
w) a DNase having at least 60%, at least 65%, at least 70%, at least 75%, at least 80%, at least 85%, at least 90%, at least 95%, at least 98% or 100% sequence identity to the amino acid sequence shown in SEQ ID NO 24,
x) a DNase having at least 60%, at least 65%, at least 70%, at least 75%, at least 80%, at least 85%, at least 90%, at least 95%, at least 98% or 100% sequence identity to the amino acid sequence shown in SEQ ID NO 25,
y) a DNase having at least 60%, at least 65%, at least 70%, at least 75%, at least 80%, at least 85%, at least 90%, at least 95%, at least 98% or 100% sequence identity to the amino acid sequence shown in SEQ ID NO 26,
z) a DNase having at least 60%, at least 65%, at least 70%, at least 75%, at least 80%, at least 85%, at least 90%, at least 95%, at least 98% or 100% sequence identity to the amino acid sequence shown in SEQ ID NO 27,
aa) a DNase having at least 60%, at least 65%, at least 70%, at least 75%, at least 80%, at least 85%, at least 90%, at least 95%, at least 98% or 100% sequence identity to the amino acid sequence shown in SEQ ID NO 28,
bb) a DNase having at least 60%, at least 65%, at least 70%, at least 75%, at least 80%, at least 85%, at least 90%,

at least 95%, at least 98% or 100% sequence identity to the amino acid sequence shown in SEQ ID NO 29,
cc) a DNase having at least 60%, at least 65%, at least 70%, at least 75%, at least 80%, at least 85%, at least 90%, at least 95%, at least 98% or 100% sequence identity to the amino acid sequence shown in SEQ ID NO 30,
dd) a DNase having at least 60%, at least 65%, at least 70%, at least 75%, at least 80%, at least 85%, at least 90%, at least 95%, at least 98% or 100% sequence identity to the amino acid sequence shown in SEQ ID NO 31,
ee) a DNase having at least 60%, at least 65%, at least 70%, at least 75%, at least 80%, at least 85%, at least 90%, at least 95%, at least 98% or 100% sequence identity to the amino acid sequence shown in SEQ ID NO 32,
ff) a DNase having at least 60%, at least 65%, at least 70%, at least 75%, at least 80%, at least 85%, at least 90%, at least 95%, at least 98% or 100% sequence identity to the amino acid sequence shown in SEQ ID NO 33,
gg) a DNase having at least 60%, at least 65%, at least 70%, at least 75%, at least 80%, at least 85%, at least 90%, at least 95%, at least 98% or 100% sequence identity to the amino acid sequence shown in SEQ ID NO 34,
hh) a DNase having at least 60%, at least 65%, at least 70%, at least 75%, at least 80%, at least 85%, at least 90%, at least 95%, at least 98% or 100% sequence identity to the amino acid sequence shown in SEQ ID NO 35,
ii) a DNase having at least 60%, at least 65%, at least 70%, at least 75%, at least 80%, at least 85%, at least 90%, at least 95%, at least 98% or 100% sequence identity to the amino acid sequence shown in SEQ ID NO 36,
jj) a DNase having at least 60%, at least 65%, at least 70%, at least 75%, at least 80%, at least 85%, at least 90%, at least 95%, at least 98% or 100% sequence identity to the amino acid sequence shown in SEQ ID NO 37,
kk) a DNase having at least 60%, at least 65%, at least 70%, at least 75%, at least 80%, at least 85%, at least 90%, at least 95%, at least 98% or 100% sequence identity to the amino acid sequence shown in SEQ ID NO 38,
ll) a DNase having at least 60%, at least 65%, at least 70%, at least 75%, at least 80%, at least 85%, at least 90%, at least 95%, at least 98% or 100% sequence identity to the amino acid sequence shown in SEQ ID NO 39,
mm) a DNase having at least 60%, at least 65%, at least 70%, at least 75%, at least 80%, at least 85%, at least 90%, at least 95%, at least 98% or 100% sequence identity to the amino acid sequence shown in SEQ ID NO 40,
nn) a DNase having at least 60%, at least 65%, at least 70%, at least 75%, at least 80%, at least 85%, at least 90%, at least 95%, at least 98% or 100% sequence identity to the amino acid sequence shown in SEQ ID NO 41,
oo) a DNase having at least 60%, at least 65%, at least 70%, at least 75%, at least 80%, at least 85%, at least 90%, at least 95%, at least 98% or 100% sequence identity to the amino acid sequence shown in SEQ ID NO 42,
pp) a DNase having at least 60%, at least 65%, at least 70%, at least 75%, at least 80%, at least 85%, at least 90%, at least 95%, at least 98% or 100% sequence identity to the amino acid sequence shown in SEQ ID NO 43,
qq) a DNase having at least 60%, at least 65%, at least 70%, at least 75%, at least 80%, at least 85%, at least 90%, at least 95%, at least 98% or 100% sequence identity to the amino acid sequence shown in SEQ ID NO 44,
rr) a DNase having at least 60%, at least 65%, at least 70%, at least 75%, at least 80%, at least 85%, at least 90%, at least 95%, at least 98% or 100% sequence identity to the amino acid sequence shown in SEQ ID NO 45,
ss) a DNase having at least 60%, at least 65%, at least 70%, at least 75%, at least 80%, at least 85%, at least 90%, at least 95%, at least 98% or 100% sequence identity to the amino acid sequence shown in SEQ ID NO 46,
tt) a DNase having at least 60%, at least 65%, at least 70%, at least 75%, at least 80%, at least 85%, at least 90%, at least 95%, at least 98% or 100% sequence identity to the amino acid sequence shown in SEQ ID NO 47,
uu) a DNase having at least 60%, at least 65%, at least 70%, at least 75%, at least 80%, at least 85%, at least 90%, at least 95%, at least 98% or 100% sequence identity to the amino acid sequence shown in SEQ ID NO 48,
vv) a DNase having at least 60%, at least 65%, at least 70%, at least 75%, at least 80%, at least 85%, at least 90%, at least 95%, at least 98% or 100% sequence identity to the amino acid sequence shown in SEQ ID NO 49,
ww) a DNase having at least 60%, at least 65%, at least 70%, at least 75%, at least 80%, at least 85%, at least 90%, at least 95%, at least 98% or 100% sequence identity to the amino acid sequence shown in SEQ ID NO 50,
xx) a DNase having at least 60%, at least 65%, at least 70%, at least 75%, at least 80%, at least 85%, at least 90%, at least 95%, at least 98% or 100% sequence identity to the amino acid sequence shown in SEQ ID NO 51,
yy) a DNase having at least 60%, at least 65%, at least 70%, at least 75%, at least 80%, at least 85%, at least 90%, at least 95%, at least 98% or 100% sequence identity to the amino acid sequence shown in SEQ ID NO 52,
zz) a DNase having at least 60%, at least 65%, at least 70%, at least 75%, at least 80%, at least 85%, at least 90%, at least 95%, at least 98% or 100% sequence identity to the amino acid sequence shown in SEQ ID NO 53,
aaa) a DNase having at least 60%, at least 65%, at least 70%, at least 75%, at least 80%, at least 85%, at least 90%, at least 95%, at least 98% or 100% sequence identity to the amino acid sequence shown in SEQ ID NO 54,
bbb) a DNase having at least 60%, at least 65%, at least 70%, at least 75%, at least 80%, at least 85%, at least 90%, at least 95%, at least 98% or 100% sequence identity to the amino acid sequence shown in SEQ ID NO 55,
ccc) a DNase having at least 60%, at least 65%, at least 70%, at least 75%, at least 80%, at least 85%, at least 90%, at least 95%, at least 98% or 100% sequence identity to the amino acid sequence shown in SEQ ID NO 56,
ddd) a DNase having at least 60%, at least 65%, at least 70%, at least 75%, at least 80%, at least 85%, at least 90%, at least 95%, at least 98% or 100% sequence identity to the amino acid sequence shown in SEQ ID NO 57,
eee) a DNase having at least 60%, at least 65%, at least 70%, at least 75%, at least 80%, at least 85%, at least

90%, at least 95%, at least 98% or 100% sequence identity to the amino acid sequence shown in SEQ ID NO 58,
fff) a DNase having at least 60%, at least 65%, at least 70%, at least 75%, at least 80%, at least 85%, at least 90%, at least 95%, at least 98% or 100% sequence identity to the amino acid sequence shown in SEQ ID NO 59,
ggg) a DNase having at least 60%, at least 65%, at least 70%, at least 75%, at least 80%, at least 85%, at least 90%, at least 95%, at least 98% or 100% sequence identity to the amino acid sequence shown in SEQ ID NO 60,
hhh) a DNase having at least 60%, at least 65%, at least 70%, at least 75%, at least 80%, at least 85%, at least 90%, at least 95%, at least 98% or 100% sequence identity to the amino acid sequence shown in SEQ ID NO 61,
iii) a DNase having at least 60%, at least 65%, at least 70%, at least 75%, at least 80%, at least 85%, at least 90%, at least 95%, at least 98% or 100% sequence identity to the amino acid sequence shown in SEQ ID NO 62,
jjj) a DNase having at least 60%, at least 65%, at least 70%, at least 75%, at least 80%, at least 85%, at least 90%, at least 95%, at least 98% or 100% sequence identity to the amino acid sequence shown in SEQ ID NO 63,
kkk) a DNase having at least 60%, at least 65%, at least 70%, at least 75%, at least 80%, at least 85%, at least 90%, at least 95%, at least 98% or 100% sequence identity to the amino acid sequence shown in SEQ ID NO 64,
lll) a DNase having at least 60%, at least 65%, at least 70%, at least 75%, at least 80%, at least 85%, at least 90%, at least 95%, at least 98% or 100% sequence identity to the amino acid sequence shown in SEQ ID NO 65,
mmm) a DNase having at least 60%, at least 65%, at least 70%, at least 75%, at least 80%, at least 85%, at least 90%, at least 95%, at least 98% or 100% sequence identity to the amino acid sequence shown in SEQ ID NO 66,
nnn) v a DNase having at least 60%, at least 65%, at least 70%, at least 75%, at least 80%, at least 85%, at least 90%, at least 95%, at least 98% or 100% sequence identity to the amino acid sequence shown in SEQ ID NO 67,
ooo) a DNase having at least 60%, at least 65%, at least 70%, at least 75%, at least 80%, at least 85%, at least 90%, at least 95%, at least 98% or 100% sequence identity to the amino acid sequence shown in SEQ ID NO 68,
ppp) a DNase having at least 60%, at least 65%, at least 70%, at least 75%, at least 80%, at least 85%, at least 90%, at least 95%, at least 98% or 100% sequence identity to the amino acid sequence shown in SEQ ID NO 69,
qqq) a DNase having at least 60%, at least 65%, at least 70%, at least 75%, at least 80%, at least 85%, at least 90%, at least 95%, at least 98% or 100% sequence identity to the amino acid sequence shown in SEQ ID NO 70,
rrr) a DNase having at least 60%, at least 65%, at least 70%, at least 75%, at least 80%, at least 85%, at least 90%, at least 95%, at least 98% or 100% sequence identity to the amino acid sequence shown in SEQ ID NO 71,
sss) a DNase having at least 60%, at least 65%, at least 70%, at least 75%, at least 80%, at least 85%, at least 90%, at least 95%, at least 98% or 100% sequence identity to the amino acid sequence shown in SEQ ID NO 72,
ttt) a DNase having at least 60%, at least 65%, at least 70%, at least 75%, at least 80%, at least 85%, at least 90%, at least 95%, at least 98% or 100% sequence identity to the amino acid sequence shown in SEQ ID NO 73,
uuu) a DNase having at least 60%, at least 65%, at least 70%, at least 75%, at least 80%, at least 85%, at least 90%, at least 95%, at least 98% or 100% sequence identity to the amino acid sequence shown in SEQ ID NO 74,
vvv) a DNase having at least 60%, at least 65%, at least 70%, at least 75%, at least 80%, at least 85%, at least 90%, at least 95%, at least 98% or 100% sequence identity to the amino acid sequence shown in SEQ ID NO 75,
www) a DNase having at least 60%, at least 65%, at least 70%, at least 75%, at least 80%, at least 85%, at least 90%, at least 95%, at least 98% or 100% sequence identity to the amino acid sequence shown in SEQ ID NO 76,
xxx) a DNase having at least 60%, at least 65%, at least 70%, at least 75%, at least 80%, at least 85%, at least 90%, at least 95%, at least 98% or 100% sequence identity to the amino acid sequence shown in SEQ ID NO 77,
yyy) a DNase having at least 60%, at least 65%, at least 70%, at least 75%, at least 80%, at least 85%, at least 90%, at least 95%, at least 98% or 100% sequence identity to the amino acid sequence shown in SEQ ID NO 78,
zzz) a DNase having at least 60%, at least 65%, at least 70%, at least 75%, at least 80%, at least 85%, at least 90%, at least 95%, at least 98% or 100% sequence identity to the amino acid sequence shown in SEQ ID NO 79,
aaaa) a DNase having at least 60%, at least 65%, at least 70%, at least 75%, at least 80%, at least 85%, at least 90%, at least 95%, at least 98% or 100% sequence identity to the amino acid sequence shown in SEQ ID NO 80,
bbbb) a DNase having at least 60%, at least 65%, at least 70%, at least 75%, at least 80%, at least 85%, at least 90%, at least 95%, at least 98% or 100% sequence identity to the amino acid sequence shown in SEQ ID NO 81,
cccc) a DNase having at least 60%, at least 65%, at least 70%, at least 75%, at least 80%, at least 85%, at least 90%, at least 95%, at least 98% or 100% sequence identity to the amino acid sequence shown in SEQ ID NO 82,
dddd) a DNase having at least 60%, at least 65%, at least 70%, at least 75%, at least 80%, at least 85%, at least 90%, at least 95%, at least 98% or 100% sequence identity to the amino acid sequence shown in SEQ ID NO 83,
eeee) a DNase having at least 60%, at least 65%, at least 70%, at least 75%, at least 80%, at least 85%, at least 90%, at least 95%, at least 98% or 100% sequence identity to the amino acid sequence shown in SEQ ID NO 84,
ffff) a DNase having at least 60%, at least 65%, at least 70%, at least 75%, at least 80%, at least 85%, at least 90%, at least 95%, at least 98% or 100% sequence identity to the amino acid sequence shown in SEQ ID NO 85,
gggg) a DNase having at least 60%, at least 65%, at least 70%, at least 75%, at least 80%, at least 85%, at least 90%, at least 95%, at least 98% or 100% sequence identity to the amino acid sequence shown in SEQ ID NO 86,
hhhh) a DNase having at least 60%, at least 65%, at least 70%, at least 75%, at least 80%, at least 85%, at least

90%, at least 95%, at least 98% or 100% sequence identity to the amino acid sequence shown in SEQ ID NO 87,
iiii)a DNase having at least 60%, at least 65%, at least 70%, at least 75%, at least 80%, at least 85%, at least 90%, at least 95%, at least 98% or 100% sequence identity to the amino acid sequence shown in SEQ ID NO 88,
jjjj)a DNase having at least 60%, at least 65%, at least 70%, at least 75%, at least 80%, at least 85%, at least 90%, at least 95%, at least 98% or 100% sequence identity to the amino acid sequence shown in SEQ ID NO 89,
kkkk) a DNase having at least 60%, at least 65%, at least 70%, at least 75%, at least 80%, at least 85%, at least 90%, at least 95%, at least 98% or 100% sequence identity to the amino acid sequence shown in SEQ ID NO 90,
llll)a DNase having at least 60%, at least 65%, at least 70%, at least 75%, at least 80%, at least 85%, at least 90%, at least 95%, at least 98% or 100% sequence identity to the amino acid sequence shown in SEQ ID NO 91,
mmmm) a DNase having at least 60%, at least 65%, at least 70%, at least 75%, at least 80%, at least 85%, at least 90%, at least 95%, at least 98% or 100% sequence identity to the amino acid sequence shown in SEQ ID NO 92,
nnnn) a DNase having at least 60%, at least 65%, at least 70%, at least 75%, at least 80%, at least 85%, at least 90%, at least 95%, at least 98% or 100% sequence identity to the amino acid sequence shown in SEQ ID NO 93,
oooo) a DNase having at least 60%, at least 65%, at least 70%, at least 75%, at least 80%, at least 85%, at least 90%, at least 95%, at least 98% or 100% sequence identity to the amino acid sequence shown in SEQ ID NO 94,
pppp) a DNase having at least 60%, at least 65%, at least 70%, at least 75%, at least 80%, at least 85%, at least 90%, at least 95%, at least 98% or 100% sequence identity to the amino acid sequence shown in SEQ ID NO 95,
qqqq) a DNase having at least 60%, at least 65%, at least 70%, at least 75%, at least 80%, at least 85%, at least 90%, at least 95%, at least 98% or 100% sequence identity to the amino acid sequence shown in SEQ ID NO 96,
rrrr) a DNase having at least 60%, at least 65%, at least 70%, at least 75%, at least 80%, at least 85%, at least 90%, at least 95%, at least 98% or 100% sequence identity to the amino acid sequence shown in SEQ ID NO 97,
ssss) a DNase having at least 60%, at least 65%, at least 70%, at least 75%, at least 80%, at least 85%, at least 90%, at least 95%, at least 98% or 100% sequence identity to the amino acid sequence shown in SEQ ID NO 98,
tttt)a DNase having at least 60%, at least 65%, at least 70%, at least 75%, at least 80%, at least 85%, at least 90%, at least 95%, at least 98% or 100% sequence identity to the amino acid sequence shown in SEQ ID NO 99,
uuuu) a DNase having at least 60%, at least 65%, at least 70%, at least 75%, at least 80%, at least 85%, at least 90%, at least 95%, at least 98% or 100% sequence identity to the amino acid sequence shown in SEQ ID NO 100,
vvvv) a DNase having at least 60%, at least 65%, at least 70%, at least 75%, at least 80%, at least 85%, at least 90%, at least 95%, at least 98% or 100% sequence identity to the amino acid sequence shown in SEQ ID NO 101, and
wwww) a DNase having at least 60%, at least 65%, at least 70%, at least 75%, at least 80%, at least 85%, at least 90%, at least 95%, at least 98% or 100% sequence identity to the amino acid sequence shown in SEQ ID NO 102.

In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC2_A DNase. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC2_A DNase, wherein the DNase is selected from the group consisting of DNases shown in Table 2. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a DNase selected from the group consisting of:

a) a DNase having at least 60%, at least 65%, at least 70%, at least 75%, at least 80%, at least 85%, at least 90%, at least 95%, at least 98% or 100% sequence identity to the amino acid sequence shown in SEQ ID NO 103,
b) a DNase having at least 60%, at least 65%, at least 70%, at least 75%, at least 80%, at least 85%, at least 90%, at least 95%, at least 98% or 100% sequence identity to the amino acid sequence shown in SEQ ID NO 104,
c) a DNase having at least 60%, at least 65%, at least 70%, at least 75%, at least 80%, at least 85%, at least 90%, at least 95%, at least 98% or 100% sequence identity to the amino acid sequence shown in SEQ ID NO 105,
d) a DNase having at least 60%, at least 65%, at least 70%, at least 75%, at least 80%, at least 85%, at least 90%, at least 95%, at least 98% or 100% sequence identity to the amino acid sequence shown in SEQ ID NO 106, and
e) a DNase having at least 60%, at least 65%, at least 70%, at least 75%, at least 80%, at least 85%, at least 90%, at least 95%, at least 98% or 100% sequence identity to the amino acid sequence shown in SEQ ID NO 107.

In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC2_B DNase. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC2_B DNase, wherein the DNase is selected from the group consisting of DNases shown in Table 3. In one embodiment, a DNase having at least 60%, at least 65%,

at least 70%, at least 75%, at least 80%, at least 85%, at least 90%, at least 95%, at least 98% or 100% sequence identity to the amino acid sequence shown in SEQ ID NO 1 is combined with a DNase selected from the group consisting of:

a) a DNase having at least 60%, at least 65%, at least 70%, at least 75%, at least 80%, at least 85%, at least 90%, at least 95%, at least 98% or 100% sequence identity to the amino acid sequence shown in SEQ ID NO 108,
b) a DNase having at least 60%, at least 65%, at least 70%, at least 75%, at least 80%, at least 85%, at least 90%, at least 95%, at least 98% or 100% sequence identity to the amino acid sequence shown in SEQ ID NO 109,
c) a DNase having at least 60%, at least 65%, at least 70%, at least 75%, at least 80%, at least 85%, at least 90%, at least 95%, at least 98% or 100% sequence identity to the amino acid sequence shown in SEQ ID NO 110,
d) a DNase having at least 60%, at least 65%, at least 70%, at least 75%, at least 80%, at least 85%, at least 90%, at least 95%, at least 98% or 100% sequence identity to the amino acid sequence shown in SEQ ID NO 111,
e) a DNase having at least 60%, at least 65%, at least 70%, at least 75%, at least 80%, at least 85%, at least 90%, at least 95%, at least 98% or 100% sequence identity to the amino acid sequence shown in SEQ ID NO 112,
f) a DNase having at least 60%, at least 65%, at least 70%, at least 75%, at least 80%, at least 85%, at least 90%, at least 95%, at least 98% or 100% sequence identity to the amino acid sequence shown in SEQ ID NO 113,
g) a DNase having at least 60%, at least 65%, at least 70%, at least 75%, at least 80%, at least 85%, at least 90%, at least 95%, at least 98% or 100% sequence identity to the amino acid sequence shown in SEQ ID NO 114,
h) a DNase having at least 60%, at least 65%, at least 70%, at least 75%, at least 80%, at least 85%, at least 90%, at least 95%, at least 98% or 100% sequence identity to the amino acid sequence shown in SEQ ID NO 115,
i) a DNase having at least 60%, at least 65%, at least 70%, at least 75%, at least 80%, at least 85%, at least 90%, at least 95%, at least 98% or 100% sequence identity to the amino acid sequence shown in SEQ ID NO 116,
j) a DNase having at least 60%, at least 65%, at least 70%, at least 75%, at least 80%, at least 85%, at least 90%, at least 95%, at least 98% or 100% sequence identity to the amino acid sequence shown in SEQ ID NO 117,
k) a DNase having at least 60%, at least 65%, at least 70%, at least 75%, at least 80%, at least 85%, at least 90%, at least 95%, at least 98% or 100% sequence identity to the amino acid sequence shown in SEQ ID NO 118,
l) a DNase having at least 60%, at least 65%, at least 70%, at least 75%, at least 80%, at least 85%, at least 90%, at least 95%, at least 98% or 100% sequence identity to the amino acid sequence shown in SEQ ID NO 119,
m) a DNase having at least 60%, at least 65%, at least 70%, at least 75%, at least 80%, at least 85%, at least 90%, at least 95%, at least 98% or 100% sequence identity to the amino acid sequence shown in SEQ ID NO 120,
n) a DNase having at least 60%, at least 65%, at least 70%, at least 75%, at least 80%, at least 85%, at least 90%, at least 95%, at least 98% or 100% sequence identity to the amino acid sequence shown in SEQ ID NO 121,
o) a DNase having at least 60%, at least 65%, at least 70%, at least 75%, at least 80%, at least 85%, at least 90%, at least 95%, at least 98% or 100% sequence identity to the amino acid sequence shown in SEQ ID NO 122,
p) a DNase having at least 60%, at least 65%, at least 70%, at least 75%, at least 80%, at least 85%, at least 90%, at least 95%, at least 98% or 100% sequence identity to the amino acid sequence shown in SEQ ID NO 123,
q) a DNase having at least 60%, at least 65%, at least 70%, at least 75%, at least 80%, at least 85%, at least 90%, at least 95%, at least 98% or 100% sequence identity to the amino acid sequence shown in SEQ ID NO 124,
r) a DNase having at least 60%, at least 65%, at least 70%, at least 75%, at least 80%, at least 85%, at least 90%, at least 95%, at least 98% or 100% sequence identity to the amino acid sequence shown in SEQ ID NO 125,
s) a DNase having at least 60%, at least 65%, at least 70%, at least 75%, at least 80%, at least 85%, at least 90%, at least 95%, at least 98% or 100% sequence identity to the amino acid sequence shown in SEQ ID NO 126,
t) a DNase having at least 60%, at least 65%, at least 70%, at least 75%, at least 80%, at least 85%, at least 90%, at least 95%, at least 98% or 100% sequence identity to the amino acid sequence shown in SEQ ID NO 127,
u) a DNase having at least 60%, at least 65%, at least 70%, at least 75%, at least 80%, at least 85%, at least 90%, at least 95%, at least 98% or 100% sequence identity to the amino acid sequence shown in SEQ ID NO 128,
v) a DNase having at least 60%, at least 65%, at least 70%, at least 75%, at least 80%, at least 85%, at least 90%, at least 95%, at least 98% or 100% sequence identity to the amino acid sequence shown in SEQ ID NO 129,
w) a DNase having at least 60%, at least 65%, at least 70%, at least 75%, at least 80%, at least 85%, at least 90%, at least 95%, at least 98% or 100% sequence identity to the amino acid sequence shown in SEQ ID NO 130,
x) a DNase having at least 60%, at least 65%, at least 70%, at least 75%, at least 80%, at least 85%, at least 90%, at least 95%, at least 98% or 100% sequence identity to the amino acid sequence shown in SEQ ID NO 131,
y) a DNase having at least 60%, at least 65%, at least 70%, at least 75%, at least 80%, at least 85%, at least 90%, at least 95%, at least 98% or 100% sequence identity to the amino acid sequence shown in SEQ ID NO 132,
z) a DNase having at least 60%, at least 65%, at least 70%, at least 75%, at least 80%, at least 85%, at least 90%, at least 95%, at least 98% or 100% sequence identity to the amino acid sequence shown in SEQ ID NO 133,
aa) a DNase having at least 60%, at least 65%, at least 70%, at least 75%, at least 80%, at least 85%, at least 90%, at least 95%, at least 98% or 100% sequence identity to the amino acid sequence shown in SEQ ID NO 134,
bb) a DNase having at least 60%, at least 65%, at least 70%, at least 75%, at least 80%, at least 85%, at least 90%,

at least 95%, at least 98% or 100% sequence identity to the amino acid sequence shown in SEQ ID NO 135,
cc) a DNase having at least 60%, at least 65%, at least 70%, at least 75%, at least 80%, at least 85%, at least 90%, at least 95%, at least 98% or 100% sequence identity to the amino acid sequence shown in SEQ ID NO 136,
dd) a DNase having at least 60%, at least 65%, at least 70%, at least 75%, at least 80%, at least 85%, at least 90%, at least 95%, at least 98% or 100% sequence identity to the amino acid sequence shown in SEQ ID NO 137,
ee) a DNase having at least 60%, at least 65%, at least 70%, at least 75%, at least 80%, at least 85%, at least 90%, at least 95%, at least 98% or 100% sequence identity to the amino acid sequence shown in SEQ ID NO 138,
ff) a DNase having at least 60%, at least 65%, at least 70%, at least 75%, at least 80%, at least 85%, at least 90%, at least 95%, at least 98% or 100% sequence identity to the amino acid sequence shown in SEQ ID NO 139,
gg) a DNase having at least 60%, at least 65%, at least 70%, at least 75%, at least 80%, at least 85%, at least 90%, at least 95%, at least 98% or 100% sequence identity to the amino acid sequence shown in SEQ ID NO 140, and
hh) a DNase having at least 60%, at least 65%, at least 70%, at least 75%, at least 80%, at least 85%, at least 90%, at least 95%, at least 98% or 100% sequence identity to the amino acid sequence shown in SEQ ID NO 141.

In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with an S1 DNase. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with an S1 DNase, wherein the DNase is selected from the group consisting of DNases shown in Table 4. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a DNase selected from the group consisting of:

a) a DNase having at least 60%, at least 65%, at least 70%, at least 75%, at least 80%, at least 85%, at least 90%, at least 95%, at least 98% or 100% sequence identity to the amino acid sequence shown in SEQ ID NO 142,
b) a DNase having at least 60%, at least 65%, at least 70%, at least 75%, at least 80%, at least 85%, at least 90%, at least 95%, at least 98% or 100% sequence identity to the amino acid sequence shown in SEQ ID NO 143,
c) a DNase having at least 60%, at least 65%, at least 70%, at least 75%, at least 80%, at least 85%, at least 90%, at least 95%, at least 98% or 100% sequence identity to the amino acid sequence shown in SEQ ID NO 144,
d) a DNase having at least 60%, at least 65%, at least 70%, at least 75%, at least 80%, at least 85%, at least 90%, at least 95%, at least 98% or 100% sequence identity to the amino acid sequence shown in SEQ ID NO 145,
e) a DNase having at least 60%, at least 65%, at least 70%, at least 75%, at least 80%, at least 85%, at least 90%, at least 95%, at least 98% or 100% sequence identity to the amino acid sequence shown in SEQ ID NO 146,
f) a DNase having at least 60%, at least 65%, at least 70%, at least 75%, at least 80%, at least 85%, at least 90%, at least 95%, at least 98% or 100% sequence identity to the amino acid sequence shown in SEQ ID NO 147,
g) a DNase having at least 60%, at least 65%, at least 70%, at least 75%, at least 80%, at least 85%, at least 90%, at least 95%, at least 98% or 100% sequence identity to the amino acid sequence shown in SEQ ID NO 148,
h) a DNase having at least 60%, at least 65%, at least 70%, at least 75%, at least 80%, at least 85%, at least 90%, at least 95%, at least 98% or 100% sequence identity to the amino acid sequence shown in SEQ ID NO 149, and
i) a DNase having at least 60%, at least 65%, at least 70%, at least 75%, at least 80%, at least 85%, at least 90%, at least 95%, at least 98% or 100% sequence identity to the amino acid sequence shown in SEQ ID NO 150.

In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC3 DNase. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a NUC3 DNase, wherein the DNase is selected from the group consisting of DNases shown in Table 5. In one embodiment, a DNase having at least 60%, at least 65%, at least 70%, at least 75%, at least 80%, at least 85%, at least 90%, at least 95%, at least 98% or 100% sequence identity to the amino acid sequence shown in SEQ ID NO 1 is combined with a DNase selected from the group consisting of:

f) a DNase having at least 60%, at least 65%, at least 70%, at least 75%, at least 80%, at least 85%, at least 90%, at least 95%, at least 98% or 100% sequence identity to the amino acid sequence shown in SEQ ID NO 151,
g) a DNase having at least 60%, at least 65%, at least 70%, at least 75%, at least 80%, at least 85%, at least 90%, at least 95%, at least 98% or 100% sequence identity to the amino acid sequence shown in SEQ ID NO 152,
h) a DNase having at least 60%, at least 65%, at least 70%, at least 75%, at least 80%, at least 85%, at least 90%, at least 95%, at least 98% or 100% sequence identity to the amino acid sequence shown in SEQ ID NO 153,
i) a DNase having at least 60%, at least 65%, at least 70%, at least 75%, at least 80%, at least 85%, at least 90%,

at least 95%, at least 98% or 100% sequence identity to the amino acid sequence shown in SEQ ID NO 154,
j) a DNase having at least 60%, at least 65%, at least 70%, at least 75%, at least 80%, at least 85%, at least 90%, at least 95%, at least 98% or 100% sequence identity to the amino acid sequence shown in SEQ ID NO 155, and
k) a DNase having at least 60%, at least 65%, at least 70%, at least 75%, at least 80%, at least 85%, at least 90%, at least 95%, at least 98% or 100% sequence identity to the amino acid sequence shown in SEQ ID NO 156.

In a preferred embodiment, DNases having different cleavage activities are combined, e.g. preferably a DNase having endo-activity is combined with a DNase having exo-activity. It is believed that the different modes of action increase the substrate removal capacity and that such DNases can act synergistically in removing DNA stains e.g. from a textile in a laundry process.

The DNases can be included in the cleaning or treatment composition of the present invention at a level of from 0.01 to 1000 ppm, from 1 ppm to 1000 ppm, from 10 ppm to 1000 ppm, from 50 ppm to 1000 ppm, from 100 ppm to 1000 ppm, from 150 ppm to 1000 ppm, from 200 ppm to 1000 ppm, from 250 ppm to 1000 ppm, from 250 ppm to 750 ppm, or from 250 ppm to 500 ppm.

The DNases above may be combined with another DNase to form a blend to be added to the wash liquor solution according to the invention. The concentration of the DNases in the wash liquor solution is typically in the range of from 0.00001 ppm to 10 ppm, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, 0.1 ppm to 10 ppm, from 0.2ppm to 10 ppm, from 0.5 ppm to 5 ppm.

One embodiment relates to a cleaning composition comprising a first DNase, a second DNase and at least one cleaning or treatment adjunct, wherein the amount of DNase in the composition is from 0.01 to 1000 ppm.

Cleaning Adjunct

The composition comprises a cleaning or treatment adjunct. Typically, the cleaning or treatment adjunct will be present in the composition in an amount from 1 to 98.9 wt%, more typically from 5 to 80 wt% cleaning adjunct. Suitable cleaning adjuncts comprise: surfactants, builders, bleach ingredients, colorants, chelating agents, dye transfer agents, deposition aids, dispersants, additional enzymes, and enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anticorrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof.

Preferably the composition comprises a surfactant. Preferably the composition comprises an anionic surfactant. Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from

0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfosuccinic acid or salt of fatty acids (soap), and combinations thereof are also suitable anionic surfactants.

The anionic surfactant is preferably added to the detergent composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or tri-ethanolamine. The composition preferably comprises from 1 to 60 weight % or from 1 to 50 wt% or 2 or 5 to 40 wt% of the composition, anionic surfactant. The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The composition of the invention preferably comprises a cleaning adjunct comprising a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac™, Lutensol™ and Pluronic™ from BASF, Dehypon™ series from Cognis and Genapol™ series from Clariant.

The detergent composition preferably comprises from 0.5wt% to about 40wt% of a non-ionic surfactant, preferably 1 to 30 wt% of the composition non-ionic surfactant.

The composition may be such that the cleaning adjunct comprises one or more selected from the group consisting of (i) perfume microcapsule; (ii) fabric hueing agent; (iii) protease; (iv) amphiphilic cleaning polymer; (v) lipase, or (vi) mixtures thereof.

The detergent composition preferably comprises one or more additional enzymes, preferably selected from the group consisting of aminopeptidase, amylase, arabinase, alginate lyase, carbohydrase, carboxypeptidase, catalase, cellulase, chitinase, cutinase, cyclodextrin glycosyltransferase, deoxyribonuclease, esterase, galactanase, alpha-galactosidase, beta-galactosidase, glucoamylase, alpha-glucosidase, beta-glucosidase, haloperoxidase, hexosaminidase, invertase, laccase, lipase, mannanase, mannosidase, oxidase such as laccase or peroxidase, pectinolytic enzyme, peptidoglutaminase, peroxidase, phytase, polyphenoloxidase, proteolytic enzyme, ribonuclease, transglutaminase, xylanase, xanthan lyase, xanthanase, endo - β-1,3-glucanase and mixtures thereof. Preferably the cleaning or treatment composition comprises additional enzyme selected from oxidase, protease, cellulase, amylase, hexosaminidase, mannanase, xanthan lyase, xanthanase, and mixtures thereof.

Preferably the composition comprises additional enzymes selected from xanthan lyase, xanthanase, mannanase, hexosaminidase and mixtures thereof. Mannanase is particularly preferred.

The additional enzyme(s) may be produced, for example, by a microorganism belonging to the genus *Aspergillus, e.g., Aspergillus aculeatus, Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum; Humicola, e.g., Humicola insolens* or *Humicola lanuginosa;* or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease, a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Preferably, the product of the invention comprises at least 0.01 mg, preferably from about 0.05 to about 10, more preferably from about 0.1 to about 6, especially from about 0.2 to about 5 mg of active further enzyme/g of composition.

Proteases: The composition of the invention can comprise one or more proteases. A mixture of two or more proteases can contribute to an enhanced cleaning across a broader temperature, cycle duration, and/or substrate range, and/or provide superior shine benefits, especially when used in conjunction with an anti-redeposition agent and/or a sulfonated polymer.

Suitable proteases for use in combination with the variant proteases of the invention include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:

subtilisins (EC 3.4.21.62), especially those derived from *Bacillus,* such as *Bacillus* sp., *B. lentus, B. alkalophilus, B. subtilis, B. amyloliquefaciens, B. gibsonii, B. akibaii, Bacillus Clausii* and *B. clarkii* described in WO2004067737, WO2015091989, WO2015091990, WO2015024739, WO2015143360, US 6,312,936 B1, US 5,679,630, US 4,760,025, DE102006022216A1, WO2015089447, WO2015089441, WO2016066756, WO2016066757, WO2016069557, WO2016069563, WO2016069569, WO2016174234, WO2017/089093, WO2020/156419. Specifically, mutations S9R, A15T, V66A, A188P, V199I, N212D, Q239R, N255D, X9E, X200L, X256E , X9R, X19L, X60D (Savinase numbering system);

subtilisins from *B. pumilus* such as the ones described in DE102006022224A1, WO2020/221578, WO2020/221579, WO2020/221580, including variants comprising amino acid substitutions in at least one or more of the positions selected from 9, 130, 133, 144, 252, 271 (BPN' numbering system);

trypsin-type or chymotrypsin-type proteases, such as trypsin (e.g., of porcine or bovine origin), including the *Fusarium* protease described in WO 89/06270 and the chymotrypsin proteases derived from *Cellumonas* described in WO 05/052161 and WO 05/052146;

metalloproteases, especially those derived from *Bacillus amyloliquefaciens* described in WO07/044993A2; from *Bacillus, Brevibacillus, Thermoactinomyces, Geobacillus, Paenibacillus, Lysinibacillus* or *Streptomyces* spp. described in WO2014194032, WO2014194054 and WO2014194117; from *Kribella alluminosa* described in WO2015193488; and from *Streptomyces* and *Lysobacter* described in WO2016075078;

protease having at least 90% identity to the subtilase from *Bacillus* sp. TY145, NCIMB 40339, described in WO92/17577 (Novozymes A/S), including the variants of this *Bacillus* sp TY145 subtilase described in WO2015024739, and WO2016066757.

Especially preferred additional proteases for the composition are polypeptides demonstrating at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the additional protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).

(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD
(viii) S9R+A15T+V68A+N218D+Q245R

Suitable commercially available additional protease enzymes include those sold under the trade names

Alcalase®, Savinase®, Primase®, Durazym®, Polarzyme®, Kannase®, Liquanase®, Liquanase Ultra®, Savinase

Ultra®, Liquanase® Evity®, Savinase® Evity®, Ovozyme®, Neutrase®, Everlase®, Coronase®, Blazed, Blaze Ultra®, Blazed Evity®, Blazed Exceed, Blaze® Pro, Esperase®, Progress® Uno, Progress® Excel, Progress® Key, Ronozyme®, Vinzon® and Het Ultra® by Novozymes A/S (Denmark);
those sold under the tradename Maxatase®, Maxacal®, Maxapem®, Properase®, Purafect®, Purafect Prime®, Purafect Ox®, FN3®, FN4®, Excellase®, Ultimase® and Purafect OXP® by Dupont;
those sold under the tradename Opticlean® and Optimase® by Solvay Enzymes;
those available from Henkel/Kemira, namely BLAP (sequence shown in Figure29 of US 5,352,604 with the following mutations S99D + S101R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and can optionally comprise a further mutation 101E or 101D;
KAP (*Bacillus alkalophilus* subtilisin with mutations A230V + S256G + S259N) from Kao; and Lavergy®, Lavergy® Pro, Lavergy® C Bright from BASF.

Especially preferred for use herein in combination with the variant protease of the invention are commercial proteases selected from the group consisting of Properase®, Blaze®, Ultimase®, Everlase®, Savinase®, Excellase®, Blaze Ultra®, BLAP and BLAP variants.

Preferred levels of protease in the product of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces*), e.g., from *H. lanuginosa* (*T. lanuginosus*) or from *H. insolens,* a *Pseudomonas lipase,* e.g., from *P. alcaligenes* or *P. pseudoalcaligenes, P. cepacia P. stutzeri, P. fluorescens, Pseudomonas* sp. strain SD 705, *P. wisconsinensis,* a *Bacillus* lipase, e.g., from *B. subtilis* (Dartois et al. (1993), Biochemica et Biophysica Acta, 1131, 253-360), *B. stearothermophilus* or *B. pumilus.*

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot 059952 (derived from *Thermomyces lanuginosus (Humicola lanuginosa)).* Preferred lipases include those sold under the tradenames Lipex®, Lipolex® and Lipoclean®.

Other suitable lipases include: Liprl 139, e.g. as described in WO2013/171241; TfuLip2, e.g. as described in WO2011/084412 and WO2013/033318; *Pseudomonas stutzeri* lipase, e.g. as described in WO2018228880; *Microbulbifer thermotolerans* lipase, e.g. as described in WO2018228881; *Sulfobacillus acidocaldarius* lipase, e.g. as described in EP3299457; LIP062 lipase e.g. as described in WO2018209026; PinLip lipase e.g. as described in WO2017036901 and *Absidia* sp. lipase e.g. as described in WO2017005798.

A suitable lipase is a variant of SEQ ID NO:5 comprising:

(a) substitution T231R
and
(b) substitution N233R or N233C
and
(c) at least three further substitutions selected from E1C, D27R, N33Q, G38A, F51V, G91Q, D96E, K98L, K98I, D111A, G163K, H198S, E210Q, Y220F, D254S, I255A, and P256T;

where the positions correspond to the positions of SEQ ID NO:5 and wherein the lipase variant has at least 90% but less than 100% sequence identity to the polypeptide having the amino acid sequence of SEQ ID NO: 5 and wherein the variant has lipase activity.

One preferred lipase is a variant of SEQ ID NO: 5 comprising the following substitutions: T231R, N233R, D27R, G38A, D96E, D111A, G163K, D254S and P256T

One preferred lipase is a variant of SEQ ID NO: 5 comprising the following substitutions: T231R, N233R, N33Q, G91Q, E210Q, I255A.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307 , US 5,648,263 , US 5,691,178, US 5,776,757 and US

5,691,178 .

In one aspect, preferred enzymes include microbial-derived endoglucanases exhibiting endo-beta-1,4-glucanase activity (E.C. 3.2.1.4), prefrebaly selected from the group comprising:

(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 246, 237, 224 and 221 of the mature polypeptide of SEQ ID NO: 2, and t he variant has cellulase activity.;
(b) a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean® and Whitezyme® (Novozymes A/S, Bagsvaerd, Denmark). Examples include Celluclean® 5000L, Celluclean® Classic 400L, Celluclean® Classic 700T, Celluclean® 4500T, Whitezyme® 1.5T, Whitezyme® 2.0L.

Other commercially available cellulases include Celluzyme®, Carezyme®, Carezyme® Premium (Novozymes A/S), Clazinase®, Puradax HA®, Revitalenz® 1000, Revitalenz® 2000 (Genencor International Inc.), KAC-500(B)® (Kao Corporation), Biotouch® FCL, Biotouch® DCL, Biotouch® DCC, Biotouch® NCD, Biotouch® FCC, Biotouch® FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:

(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183* and G184*.
(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.
(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, 1206, M208, D209, E212, V213, V214, N214, L217, R218, N219, V222, T225, T227, G229, 1235, K242, Y243, S244, F245, T246, 1250, S255, A256, H286, V291, T316, V317, V318, N417, T418, A419, H420, P421, 1428, M429, F440, R443, N444, K445, Q448, S451, A465, N470, S472.
(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.
(e) variants described in WO10/115021, especially those exhibiting at least 75%, or at least 85% or at least 90% or

at least 95% with SEQ ID NO:2 in WO10/115021, the alpha-amylase derived from *Bacillus sp.* TS-23.

(f) variants exhibiting at least 89% identity with SEQ ID NO:1 in WO2016091688, especially those comprising deletions at positions H183+G184 and additionally one or more mutations at positions 405, 421, 422 and/or 428.

(g) variants described in WO2014099523, especially those exhibiting at least 60% amino acid sequence identity with the "PcuAmyl $\alpha$-amylase" from *Paenibacillus curdlanolyticus* YK9 (SEQ ID NO:3 in WO2014099523).

(h) variants described in WO2014099523, especially those exhibiting at least 60% amino acid sequence identity with the "CspAmy2 amylase" from *Cytophaga sp.* (SEQ ID NO: 1 & 6 in WO2014164777. Especially those comprising one of more of the following deletions and/or mutations based on SEQ ID NO:1 in WO2014164777: R178*, G179*, T38N, N88H, N126Y, T129I, N134M, F153W, L171R, T180D, E187P, I203Y, G476K, G477E, Y303D.

(i) variants exhibiting at least 85% identity with AmyE from *Bacillus subtilis* (SEQ ID NO:1 in WO2009149271).

(j) variants exhibiting at least 90% identity with the wild-type amylase from Bacillus sp. KSM-K38 with accession number AB051102.

(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H*, N54S, V56T, K72R, G109A, F113Q, R116Q, W167F, Q172G, A174S, G184T, N195F, V206L, K391A, P473R, G476K.

(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL®, ATLANTIC®, ACHIEVE ALPHA®, AMPLIFY® PRIME, INTENTSA® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS®, POWERASE®, PREFERENZ S® series (including PREFERENZ S1000® and PREFERENZ S2000® and PURASTAR OXAM® (DuPont., Palo Alto, California) and KAM® (Kao, 14-10 Nihonbashi Kayabacho, 1-chome, Chuoku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from *C. cinereus,* and variants thereof as those described in WO 93/24618, WO 95/10602, and WO 98/15257.

Commercially available peroxidases include GUARDZYME® (Novozymes A/S).

Pectate lyase: Suitable pectate lyases include those sold under the tradenames Pectawash®, Pectaway®, X-Pect®, (all Novozymes A/S, Bagsvaerd, Denmark) Preferenz® F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway® (Novozymes A/S) such as Mannaway® 200L and Mannaway Evity 4.0T Other commercially available mannanases include Effectenz® M1000, Mannastar® 375, Preferenz M100 and Purabrite® (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

RNase: suitable RNases include wild-types and variants defined by SEQ ID NOS: 3, 6, 9, 12, 15, 57, 58, 59, 60, 61, 62, 63, 64, 65, 66, 67, 72 and 73 in WO2018178061 (Novozymes), incorporated herein by reference.

Hexosaminidases: The composition may comprise one or more hexosaminidases. The term hexosaminidase includes "dispersin" and the abbreviation "Dsp", which means a polypeptide having hexosaminidase activity, EC 3.2.1 .- that catalyzes the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 may be preferred, especially the variants with improved thermostability disclosed in that publication.

Galactanase: The composition may comprise a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the detergent composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable dyes include azo, anthraquinone, triarylmethane and azine dyes, azo dyes are particularly preferred, particularly bis-azo dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in

EP1794275 or EP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint® Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites, useable in laundry preferably has the formula $Na_{12}(AlO_2)_{12}(SiO_2)_{12}\cdot 27H_2O$ and the particle size is usually between 1-10 $\mu$m for zeolite A and 0.7-2 um for zeolite MAP. Other builders are Sodium metasilicate ($Na_2SiO_3 \cdot nH_2O$ or $Na_2Si_2O_5 \cdot n\ H_2O$) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers

and amphiphilic polymers and mixtures thereof.

Amphiphilic cleaning polymers: Preferably, the amphiphilic cleanimg polymer is a compound having the following general structure: bis(($C_2H_5O$)($C_2H_4O$)n)($CH_3$)-$N^+$-$C_xH_{2x}$-$N^+$-($CH_3$)-bis(($C_2H_5O$)($C_2H_4O$)n), wherein n = from 20 to 30, and x = from 3 to 8, or sulphated or sulphonated variants thereof.

Amphiphilic alkoxylated grease cleaning polymers of the present invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV):

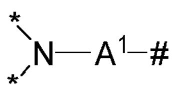
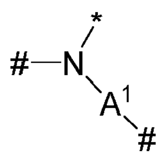
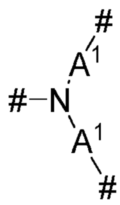
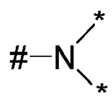

(I) (II) (III) (IV)

wherein # in each case denotes one-half of a bond between a nitrogen atom and the free binding position of a group $A^1$ of two adjacent repeating units of formulae (I), (II), (III) or (IV); * in each case denotes one-half of a bond to one of the alkoxylate groups; and $A^1$ is independently selected from linear or branched $C_2$-$C_6$-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b),

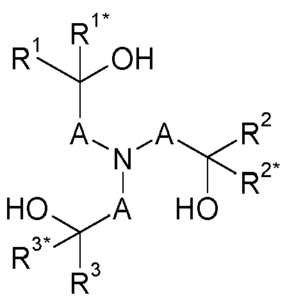
(I.a)

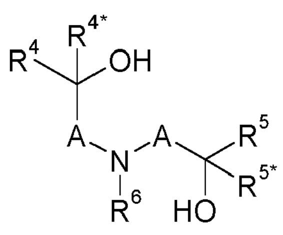
(I.b)

wherein A are independently selected from $C_1$-$C_6$-alkylene; $R^1$, $R^{1*}$, $R^2$, $R^{2*}$, $R^3$, $R^{3*}$, $R^4$, $R^{4*}$, $R^5$ and $R^{5*}$ are independently selected from hydrogen, alkyl, cycloalkyl or aryl, wherein the last three mentioned radicals may be optionally substituted; and $R^6$ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V)

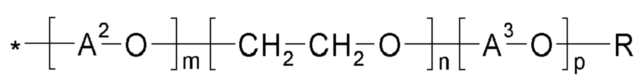

(V)

wherein * in each case denotes one-half of a bond to the nitrogen atom of the repeating unit of formula (I), (II) or (IV); $A^2$ is in each case independently selected from 1,2-propylene, 1,2-butylene and 1,2-isobutylene; $A^3$ is 1,2-propylene; R is in each case independently selected from hydrogen and $C_1$-$C_4$-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers. Preferred are those having a structure as defined by one of the following structures (I), (II) or (III):

(I) $-[(OCHR^1-CHR^2)_a-O-OC-Ar-CO-]_d$

(II) $-[(OCHT^3-CHR^4)_b-O-OC-sAr-CO-]_e$

(III) $-[(OCHR^5-CHR^6)_c-OR^7]_f$

wherein:

a, b and c are from 1 to 200;
d, e and f are from 1 to 50;
Ar is a 1,4-substituted phenylene;
sAr is 1,3-substituted phenylene substituted in position 5 with $SO_3Me$;
Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
$R^1$, $R^2$, $R^3$, $R^4$, $R^5$ and $R^6$ are independently selected from H or $C_1$-$C_{18}$ n- or iso-alkyl; and
$R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning or treatment composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g. fabric conditioners

including clays, foam boosters, suds suppressors, anti-corrosion agents, soil-suspending agents, anti-soil re-deposition agents, dyes, bactericides, optical brighteners/fluorescent whitening agents (these terms are used interchangeably), hydrotropes, tarnish inhibitors, organic solvents such as ethanol or perfumes.

Method of Use

The present invention also provides a method for treating a surface, preferably a fabric surface, the method comprising in a contacting step, contacting a surface with an aqueous wash liquor comprising a first DNase and a second DNase as described herein, preferably each in an amount from 0.00005ppm to 10ppm, preferably from 0.001ppm to 1ppm; and a cleaning adjunct.

A preferred cleaning adjunct comprises an anionic surfactant preferably in an amount from 0.05 to 50g/l, more preferably from 0.2g/l to 5g/l or 0.5g/l to 3g/l.

The aqueous wash liquor may be formed by adding a detergent composition as described above to water, for example in a washing machine or hand washing process. The concentration of detergent composition is typically from 500ppm to 15000ppm, preferably from 1000 to 10000ppm, preferably from 1000 to 5000ppm.

Alternatively, the aqueous wash liquor may be formed by adding the first DNase and the second DNAse and cleaning adjunct as separate components, into water to form the wash liquor. The surface, preferably fabric may be optionally subsequently washed, and/or rinsed and/or dried.

In the contacting step, or in a subsequent step, it may be preferred to use mechanical agitation to promote cleaning and removal of the broken-down soil by-products from the fabric. The wash liquor preferably has a pH of from about 7 or 8 to about 10.5. The wash liquor preferably has a temperature from about 5 °C to about 40 °C, or preferably from 10 to 30 °C or less than 30 °C. The water to fabric ratio is typically from about 1:1 to about 30:1.

The pH of the wash liquor is typically in the range about 5.5 to about 10, more typically in the range of 7 to 9, such as in the range of about 7 to about 8.5 or about 7 to about 8.

The concentration of each enzyme (first DNase, second DNase and any additional enzyme), in the wash liquor is typically in the range of from 0.00001 ppm to 10 ppm enzyme protein, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, from 0.1 ppm to 10 ppm, from 0.2 ppm to 10 ppm, or from 0.5 ppm to 5 ppm.

Definitions

The term "DNase" means a polypeptide having DNase activity that catalyzes the hydrolytic cleavage of phosphodiester linkages in a DNA backbone, thus degrading DNA. The term "DNases" and the expression "a polypeptide with DNase activity" are used interchangeably throughout the application. For purposes of the present invention, DNase activity may be determined according to the procedure described in Assay I. Preferably the DNase is selected from any of the enzyme classes E.C. 3.1.21.X, where X = 1, 2, 3, 4, 5, 6, 7, 8 or 9, e.g. Deoxyribonuclease I, Deoxyribonuclease IV, Type I site-specific deoxyribonuclease, Type II site-specific deoxyribonuclease, Type III site-specific deoxyribonuclease, CC-preferring endo-deoxyribonuclease, Deoxyribonuclease V, T(4) deoxyribonuclease II, T(4) deoxyribonuclease IV or E.C. 3.1.22.Y where Y = 1, 2, 4 or 5, e.g. Deoxyribonuclease II, Aspergillus deoxyribonuclease K(1), Crossover junction endo-deoxyribonuclease, Deoxyribonuclease X.

Preferably, the polypeptide having DNase activity is obtained from a microorganism and the DNase is a microbial enzyme.

The term "bacterial" as used herein in relation to DNase polypeptide refers to both polypeptides encoded by and thus directly derivable from the genome of a bacteria, as well as to genetically modified variants of such polypeptides. In the context of the present invention, the terms "bacterial DNase", "DNase obtained/obtainable from a bacterial source" or "polypeptide having DNase activity obtained/obtainable from a bacterial source" thus refer to both wildtype bacterial DNase polypeptides and genetically modified variants thereof. In a further aspect, the invention includes polypeptides having DNase activity, wherein said polypeptides are substantially homologous to a bacterial DNase. In the context of the present invention, the term "substantially homologous" denotes a polypeptide having DNase activity which is at least 80%, preferably at least 85%, more preferably at least 90%, such as at least 95%, at least 96%, at least 97%, at least 98% or at least 99% identical to the amino acid sequence of a selected bacterial DNase. The bacterial DNase may be combined with a fungal DNase or another bacterial DNase and included in the cleaning or treatment composition s and methods of the present invention.

The term "biofilm" means a substance produced by any group of microorganisms in which cells stick to each other or stick to a surface, such as a textile, dishware or hard surface or another kind of surface. These adherent cells are frequently embedded within a self-produced matrix of extracellular polymeric substance (EPS). Biofilm EPS is a polymeric conglomeration generally composed of extracellular DNA, RNA, proteins, and polysaccharides. Biofilms may form on living or non-living surfaces. The microbial cells growing in a biofilm are physiologically distinct from planktonic

cells of the same organism, which, by contrast, are single cells that may float or swim in a liquid medium. Bacteria living in a biofilm usually have significantly different properties from planktonic bacteria of the same species, as the dense and protected environment of the film allows them to cooperate and interact in various ways. One benefit of this environment for the microorganisms is increased resistance to detergents and antibiotics, as the dense extracellular matrix and the outer layer of cells protect the interior of the community. On laundry, biofilm-producing bacteria can be found among the following species: *Acinetobacter sp., Aeromicrobium sp., Brevundimonas sp., Microbacterium sp., Micrococcus luteus, Pseudomonas sp., Staphylococcus epidermidis, and Stenotrophomonas sp.* On hard surfaces, biofilm-producing bacteria can be found among the following species: *Acinetobacter sp., Aeromicrobium sp., Brevundimonas sp., Microbacterium sp., Micrococcus luteus, Pseudomonas sp., Staphylococcus epidermidis, Staphylococcus aureus and Stenotrophomonas sp.* In one aspect, the biofilm-producing strain is *Brevundimonas sp.* In one aspect, the biofilm-producing strain is *Pseudomonas alcaliphila* or *Pseudomonas fluorescens.*

The term "cleaning or treatment adjunct", e.g. a detergent adjunct ingredient, is different from the at least two DNase enzymes. The precise nature of these additional cleaning or treatment adjuncts e.g. adjunct components, and levels of incorporation thereof, will depend on the physical form of the composition and the nature of the operation for which it is to be used. Suitable cleaning or treatment adjuncts e.g. adjunct materials include, but are not limited, to the components described above such as surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric agents, clay soil removal/anti-redeposition agents, brighteners, suds suppressors, dyes, perfumes, structure elasticizing agents, fabric softeners, carriers, hydrotropes, builders and co-builders, fabric huing agents, anti-foaming agents, dispersants, processing aids, and/or pigments.

The term "cleaning composition" refers to compositions that find use in the removal of undesired compounds from items to be cleaned, such as textiles or surfaces such as hard surfaces. The cleaning composition may be used to e.g. clean textiles for both household cleaning and industrial cleaning. The term encompasses any materials/compounds selected for the particular type of cleaning composition desired and the form of the product (e.g., liquid, gel, powder, granulate, paste, or spray compositions) and includes, but is not limited to, detergent compositions (e.g., liquid and/or solid laundry detergents and fine fabric detergents; and fabric/textile and laundry pre-spotters/pretreatment). Treatment compositions comprise for example, fabric fresheners; fabric softeners. In addition to containing the DNases, the cleaning or treatment composition may contain one or more additional enzymes, such as amylases, proteases, lipases, cellulases, xyloglucanases, mannanases, pectate lyases, perhydrolases, peroxidases, lipoxygenases, laccases, hemicellulases, cellulases, cellobiose dehydrogenases, xylanases, phospho lipases, esterases, cutinases, pectinases, keratinases, reductases, oxidases, phenoloxidases, ligninases, pullulanases, tannases, pentosanases, lichenases glucanases, arabinosidases, hyaluronidase, chondroitinase and mixtures thereof, and/or cleaning or treatment adjunct e.g. detergent adjunct ingredients such as surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

The term "fungal" as used herein in relation to a DNase polypeptide refers to both polypeptides encoded by and thus directly derivable from the genome of a fungus, as well as to genetically modified variants of such polypeptides. In the context of the present invention, the terms "fungal DNase", "DNase obtained/obtainable from a fungal source" or "polypeptide having DNase activity obtained/obtainable from a fungal source" thus refer to both wildtype fungal DNase polypeptides and to genetically modified variants thereof. In a further aspect, the invention includes polypeptides having DNase activity, wherein said polypeptides are substantially homologous to a fungal DNase. In the context of the present invention, the term "substantially homologous" denotes a polypeptide having DNase activity which is at least 80%, preferably at least 85%, more preferably at least 90%, such as at least 95%, at least 96%, at least 97%, at least 98% or at least 99% identical to the amino acid sequence of a selected fungal DNase. The fungal DNase may be combined with another or a second fungal DNase or a bacterial DNase and included in the cleaning or treatment compositions and methods of the present invention.

The term "hard surface cleaning" is defined herein as cleaning of hard surfaces wherein hard surfaces may include floors, tables, walls, roofs etc. as well as surfaces of hard objects such as cars (car wash) and dishes (dishwashing). Dishwashing includes but are not limited to cleaning of plates, cups, glasses, bowls, cutlery such as spoons, knives, forks, serving utensils, ceramics, plastics, metals, china, glass and acrylics.

The term "laundering" relates to both household laundering and industrial laundering and means the process of treating textiles/fabrics with a solution containing a cleaning e.g. detergent composition of the present invention. The laundering process can for example be carried out using e.g. a household or an industrial washing machine or can be carried out by hand.

By the term "malodor" is meant an odor which is not desired on clean items. The cleaned item should smell fresh and clean without malodors adhered to the item. One example of malodor is compounds with an unpleasant smell

which may be associated with biofilm, sebum, dead cell material and similar organic soil. Another example of unpleasant smells can be sweat or body odor adhered to an item, which has been in contact with human or animal. Another example of malodor can be the odor from smoke, pollution or spices, which sticks to items for example curry or other exotic spices which smell strongly.

The term "textile" means any textile material including yarns, yarn intermediates, fibres, non-woven materials, natural materials, synthetic materials, and any other textile material, fabrics made of these materials and products made from fabrics (e.g., garments and other articles). The textile or fabric may be for example in the form of knits, wovens, denims, non-wovens, felts, yarns, and towelling. The textile or fabric may be cellulose based such as natural cellulosics, including cotton, flax/linen, jute, ramie, sisal or coir or manmade cellulosics (e.g. originating from wood pulp) including viscose/rayon, cellulose acetate fibers (tricell), lyocell or blends thereof. The textile or fabric may also be non-cellulose based such as natural polyamides including wool, camel, cashmere, mohair, rabbit and silk or synthetic polymers such as nylon, aramid, polyester, acrylic, polypropylene and spandex/elastane, or blends thereof as well as blends of cellulose based and non-cellulose based fibers. Examples of blends are blends of cotton and/or rayon/viscose with one or more companion material such as wool, synthetic fiber (e.g. polyamide fiber, acrylic fiber, polyester fiber, polyvinyl chloride fiber, polyurethane fiber, polyurea fiber, aramid fiber), and/or cellulose-containing fiber (e.g. rayon/viscose, ramie, flax/linen, jute, cellulose acetate fiber, lyocell). Fabric may be conventional washable laundry, for example stained household laundry. When the term fabric or garment is used, it is intended to include the broader term textiles as well.

The term "variant" means a polypeptide having the activity of the parent or precursor polypeptide and comprising an alteration, i.e., a substitution, insertion, and/or deletion, at one or more positions compared to the precursor or parent polypeptide. A substitution means replacement of the amino acid occupying a position with a different amino acid; a deletion means removal of the amino acid occupying a position; and an insertion means adding an amino acid adjacent to and immediately following the amino acid occupying a position.

The term "wash performance" is used as an enzyme's ability to remove stains present on the object to be cleaned during e.g. wash or hard surface cleaning.

The term "wash liquor" refers to a mixture of water and a cleaning or treatment composition of the invention used for washing objects to be cleaned, e.g. textiles or dishes.

The term "whiteness" is defined herein as a greying, yellowing of a textile. Loss of whiteness may be due to removal of optical brighteners/hueing agents. Greying and yellowing can be due to soil redeposition, body soils, colouring from e.g. iron and copper ions or dye transfer. Whiteness might include one or several issues from the list below: colourant or dye effects; incomplete stain removal (e.g. body soils, sebum etc.); redeposition (greying, yellowing or other discolourations of the object) (removed soils reassociate with other parts of textile, soiled or unsoiled); chemical changes in textile during application; and clarification or brightening of colours.

## Sequence identity

The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues x } 100)/(\text{Length of Alignment} - \text{Total Number of Gaps in Alignment})$$

## Nomenclature

For purposes of the present invention, the nomenclature [E/Q] or [EQ] means that the amino acid at this position may be a glutamic acid (Glu, E) or a glutamine (Gln, Q). Likewise, the nomenclature [V/G/A/I] or [VGAI] means that the amino acid at this position may be a valine (Val, V), glycine (Gly, G), alanine (Ala, A) or isoleucine (Ile, I), and so forth for other combinations as described herein. Unless otherwise limited further, the amino acid X is defined such that it may be any of the 20 natural amino acids.

EXAMPLES

Assays

Assay I: testing of DNase activity

DNase activity may be determined by fluorescence using a fluorescence-quenched DNA oligonucleotide probe. This probe emits a signal after nuclease degradation according to the manual from the supplier (DNase alert kit, Integrated DNA Technology, Coralville, Iowa, USA). Briefly, 5μl of the substrate is added to 95 μl of DNase. If the signal is too high, further dilutions of DNase are performed in a suitable buffer. Kinetic curves are measured for 20 min at 22°C using a Clariostar microplate reader (536 nm excitation, 556 nm emission).

Example 1

Preparation of pillowcase extract:

Extracts of real items - heavy soiled pillowcases from Warwick Equest

2 g of swatches (0.5 cm x 0.5 cm) from the middle of a heavy soiled pillowcase were placed into a 50 ml tube and 30 ml of extraction buffer (0.9 (w/w) % NaCl; 10 mM EDTA) were added and the tube was placed in a Stuart rotator for 60 min at 40 rpm. This was done for three pillowcases. Afterwards, tubes were centrifuged 4000 rpm for 10 min at 20°C to remove textile. Supernatants were collected from each tube and pooled. Supernatant was further clarified by filtration through a 0.2 μm filter.

Measurement of boosting effects:

Residual DNA in extracts was measured with a Quant-iT™ PicoGreen™ dsDNA Assay Kit (P7589; ThermoFisher Scientific) using a fluorometer (CLARIOstar Omega Clariostar Omega (BMG Labtech, Ortenberg, Germany).
The following solutions were prepared:

Table 6

| Name | Procedure | Storage |
|---|---|---|
| 1xTE buffer | 20-fold dilution of the TE buffer provided in kit (e.g. 5mL TE buffer to 95mL sterile DNase-free water) | Up to 6 months at room temperature |
| Assay reagent | 200-fold dilution of concentrated reagent from kit. (e.g. 100μL reagent to 20mL 1xTE buffer) | Pack in tinfoil, use within hours. |
| 2x15°dH water | Prepare 1L with 4:1 Ca/Mg ratio:<br>1. Add 0.674g NaHCO3 and a magnet to a jug<br>2. Add 1000mL Milli-Q water<br>3. Add 6.0mL 4000°dH CaCl2 stock<br>4. Add 3.0mL MgCl2 stock<br>5. Stir for a minimum of 10min | Prepare and use on the day of the experiment. |

In a well of a black microtiter plate, 50μl of pillowcase extract was adjusted resulting in water hardness 15°dH, and 100μl picogreen (dilution 200x) was added. Enzymes were added resulting in a concentration of 0.2 ppm and incubated for 1h at room temperature. Fluorescence was measured at excitation 483-15nm/emmision530-30nm.
The results are shown in Table 7 below, where a lower number indicates less DNA staining.

Table 7

| Column 1 (Enzyme sequence) | Organisms | First DNase (SEQ ID NO 1) | First DNase (SEQ ID NO 1) + Second DNase (Column 1) | Class of DNase |
|---|---|---|---|---|
| SEQ ID NO 77 | Fungi | 77 | 55 | NUC1_A |
| SEQ ID NO 41 | Fungi | 77 | 56 | NUC1_A |

(continued)

| Column 1 (Enzyme sequence) | Organisms | First DNase (SEQ ID NO 1) | First DNase (SEQ ID NO 1) + Second DNase (Column 1) | Class of DNase |
|---|---|---|---|---|
| SEQ ID NO 13 | Fungi | 77 | 56 | NUC1_A |
| SEQ ID NO 64 | Fungi | 77 | 60 | NUC1_A |
| SEQ ID NO 49 | Fungi | 77 | 60 | NUC1_A |
| SEQ ID NO 21 | Fungi | 77 | 61 | NUC1_A |
| SEQ ID NO 80 | Fungi | 77 | 62 | NUC1_A |
| SEQ ID NO 88 | Fungi | 77 | 64 | NUC1_A |
| SEQ ID NO 38 | Fungi | 77 | 64 | NUC1_A |
| SEQ ID NO 90 | Fungi | 77 | 65 | NUC1_A |
| SEQ ID NO 51 | Fungi | 77 | 65 | NUC1_A |
| SEQ ID NO 72 | Fungi | 77 | 65 | NUC1_A |
| SEQ ID NO 76 | Fungi | 77 | 66 | NUC1_A |
| SEQ ID NO 92 | Fungi | 77 | 66 | NUC1_A |
| SEQ ID NO 37 | Fungi | 77 | 67 | NUC1_A |
| SEQ ID NO 98 | Fungi | 77 | 67 | NUC1_A |
| SEQ ID NO 28 | Fungi | 77 | 67 | NUC1_A |
| SEQ ID NO 94 | Fungi | 77 | 67 | NUC1_A |
| SEQ ID NO 2 | Fungi | 77 | 68 | NUC1_A |
| SEQ ID NO 25 | Fungi | 77 | 69 | NUC1_A |
| SEQ ID NO 32 | Fungi | 77 | 69 | NUC1_A |
| SEQ ID NO 41 | Fungi | 77 | 68 | NUC1_A |
| SEQ ID NO 60 | Fungi | 77 | 70 | NUC1_A |
| SEQ ID NO 54 | Fungi | 77 | 70 | NUC1_A |
| SEQ ID NO 91 | Fungi | 77 | 70 | NUC1_A |
| SEQ ID NO 79 | Fungi | 77 | 71 | NUC1_A |
| SEQ ID NO 95 | Fungi | 77 | 72 | NUC1_A |
| SEQ ID NO 14 | Fungi | 77 | 72 | NUC1_A |
| SEQ ID NO 56 | Fungi | 77 | 72 | NUC1_A |
| SEQ ID NO 55 | Fungi | 77 | 73 | NUC1_A |
| SEQ ID NO 50 | Fungi | 77 | 73 | NUC1_A |
| SEQ ID NO 11 | Fungi | 77 | 74 | NUC1_A |
| SEQ ID NO 81 | Fungi | 77 | 75 | NUC1_A |
| SEQ ID NO 67 | Bacteria | 77 | 75 | NUC1_A |
| SEQ ID NO 42 | Fungi | 77 | 75 | NUC1_A |
| SEQ ID NO 75 | Bacteria | 77 | 75 | NUC1_A |
| SEQ ID NO 15 | Bacteria | 77 | 76 | NUC1_A |
| SEQ ID NO 33 | Fungi | 77 | 76 | NUC1_A |
| SEQ ID NO 16 | Bacteria | 77 | 76 | NUC1_A |

(continued)

| Column 1 (Enzyme sequence) | Organisms | First DNase (SEQ ID NO 1) | First DNase (SEQ ID NO 1) + Second DNase (Column 1) | Class of DNase |
|---|---|---|---|---|
| SEQ ID NO 46 | Fungi | 77 | 76 | NUC1_A |
| SEQ ID NO 97 | Fungi | 77 | 76 | NUC1_A |

Table 7 shows that addition of two DNases provide an improved stain removal compared to having one DNase as the lower number indicates less DNA staining.

Table 8. Boosting on Pillowcase extracts of NUC2_A

| Column 1 (Enzyme sequence) | Organisms | First DNase (SEQ ID NO 1) | First DNase (SEQ ID NO 1) + Second DNase (Column 1) | Additional DNA removal for combination first DNase + second DNase | Class of DNase |
|---|---|---|---|---|---|
| SEQ ID NO 107 | Fungi | 75 | 34 | 41 | NUC2_A |
| SEQ ID NO 105 | Fungi | 75 | 65 | 10 | NUC2_A |
| SEQ ID NO 106 | Fungi | 75 | 66 | 9 | NUC2_A |
| SEQ ID NO 103 | Fungi | 75 | 68 | 7 | NUC2_A |
| SEQ ID NO 104 | Fungi | 75 | 70 | 5 | NUC2_A |

Table 9. Boosting on Pillowcase extracts of NUC2_B

| Column 1 (Enzyme sequence) | Organisms | First DNase (SEQ ID NO 1) | First DNase (SEQ ID NO 1) + Second DNase (Column 1) | Additional DNA removal for combination first DNase + second DNase | Class of DNase |
|---|---|---|---|---|---|
| SEQ ID NO 130 | Fungi | 75 | 17 | 57 | NUC2_B |
| SEQ ID NO 126 | Fungi | 75 | 17 | 57 | NUC2_B |
| SEQ ID NO 141 | Fungi | 75 | 18 | 56 | NUC2_B |
| SEQ ID NO 112 | Fungi | 75 | 19 | 56 | NUC2_B |
| SEQ ID NO 131 | Fungi | 75 | 19 | 55 | NUC2_B |
| SEQ ID NO 122 | Fungi | 75 | 19 | 55 | NUC2_B |
| SEQ ID NO 129 | Fungi | 75 | 20 | 55 | NUC2_B |
| SEQ ID NO 138 | Fungi | 75 | 20 | 55 | NUC2_B |
| SEQ ID NO 114 | Fungi | 75 | 20 | 54 | NUC2_B |
| SEQ ID NO 113 | Fungi | 75 | 21 | 54 | NUC2_B |
| SEQ ID NO 132 | Fungi | 75 | 21 | 53 | NUC2_B |
| SEQ ID NO 125 | Fungi | 75 | 22 | 53 | NUC2_B |
| SEQ ID NO 109 | Fungi | 75 | 22 | 53 | NUC2_B |
| SEQ ID NO 139 | Fungi | 75 | 25 | 50 | NUC2_B |
| SEQ ID NO 115 | Fungi | 75 | 26 | 49 | NUC2_B |
| SEQ ID NO 135 | Fungi | 75 | 27 | 47 | NUC2_B |
| SEQ ID NO 119 | Fungi | 75 | 27 | 47 | NUC2_B |
| SEQ ID NO 128 | Fungi | 75 | 27 | 47 | NUC2_B |
| SEQ ID NO 127 | Fungi | 75 | 27 | 47 | NUC2_B |

(continued)

| Column 1 (Enzyme sequence) | Organisms | First DNase (SEQ ID NO 1) | First DNase (SEQ ID NO 1) + Second DNase (Column 1) | Additional DNA removal for combination first DNase + second DNase | Class of DNase |
|---|---|---|---|---|---|
| SEQ ID NO 133 | Fungi | 75 | 27 | 47 | NUC2_B |
| SEQ ID NO 123 | Fungi | 75 | 27 | 47 | NUC2_B |
| SEQ ID NO 120 | Fungi | 75 | 28 | 47 | NUC2_B |
| SEQ ID NO 111 | Fungi | 75 | 28 | 46 | NUC2_B |
| SEQ ID NO 110 | Fungi | 75 | 28 | 46 | NUC2_B |
| SEQ ID NO 116 | Fungi | 75 | 30 | 44 | NUC2_B |
| SEQ ID NO 118 | Fungi | 75 | 32 | 43 | NUC2_B |
| SEQ ID NO 121 | Fungi | 75 | 36 | 38 | NUC2_B |
| SEQ ID NO 136 | Fungi | 75 | 47 | 28 | NUC2_B |
| SEQ ID NO 140 | Fungi | 75 | 50 | 24 | NUC2_B |
| SEQ ID NO 134 | Fungi | 75 | 57 | 18 | NUC2_B |
| SEQ ID NO 117 | Fungi | 75 | 59 | 16 | NUC2_B |
| SEQ ID NO 108 | Fungi | 75 | 66 | 8 | NUC2_B |
| SEQ ID NO 124 | Fungi | 75 | 66 | 8 | NUC2_B |
| SEQ ID NO 137 | Fungi | 75 | 69 | 5 | NUC2_B |

Table 10. Boosting on Pillowcase extracts of S1P1

| Column 1 (Enzyme sequence) | organisms | First DNase (SEQ ID NO 1) | First DNase (SEQ ID NO 1) + Second DNase (Column 1) | Additional DNA removal for combination first DNase + second DNase | Class of DNase |
|---|---|---|---|---|---|
| SEQ ID NO 148 | Fungi | 68 | 17 | 51 | S1P1 |
| SEQ ID NO 150 | Fungi | 68 | 23 | 45 | S1P1 |
| SEQ ID NO 146 | Fungi | 68 | 26 | 42 | S1P1 |
| SEQ ID NO 145 | Fungi | 68 | 32 | 36 | S1P1 |
| SEQ ID NO 147 | Fungi | 68 | 35 | 33 | S1P1 |
| SEQ ID NO 142 | Fungi | 68 | 41 | 27 | S1P1 |
| SEQ ID NO 144 | Fungi | 68 | 45 | 23 | S1P1 |
| SEQ ID NO 143 | Fungi | 68 | 54 | 14 | S1P1 |
| SEQ ID NO 149 | Fungi | 68 | 63 | 5 | S1P1 |

Example 2

Construction of clades and phylogenetic trees

NUC1

The NUC1_A nucleases comprise the domain DUF1524, as defined in PFAM (PF07510, Pfam version 30.0 Finn (2016). Nucleic Acids Research, Database Issue 44:D279-D285). The sequences were aligned using the MUSCLE algorithm version 3.8.31 (Edgar, 2004. Nucleic Acids Research 32(5): 1792-1797.

The polypeptides in DUF1524 can be separated into distinct sub-clusters, where we denoted one sub-cluster

comprising the motif [F/L/Y/I]A[N/R]D[L/I/P/V][ (SEQ ID NO 158) as family NUC1. The motif is located at positions corresponding to positions 111 to 115 of SEQ ID NO 1. Another motif characteristic of this domain is C[D/N]T[A/R] (SEQ ID NO 159), located at positions corresponding to positions 44 to 47 of (SEQ ID NO 1).

The polypeptides in NUC1 can be separated into distinct sub-clusters, one of which was denoted NUC1_A. A characteristic motif for this subgroup is the motif [DQ][IV]D[H] (SEQ ID NO 160) corresponding to amino acids 85 to 88 in the reference polypeptide (SEQ ID NO: 1). The D at the position corresponding to position 85 of SEQ ID NO 21 is predicted to be involved in binding of catalytic metal ion cofactor. The nucleases shown in SEQ ID Nos: 1 to 102 are defined as NUC1_A DNases.

NUC2

A subgroup of Exo-endo-phos (Pfam domain id PF03372, Pfam version 31.0 Finn (2016). Nucleic Acids Research, Database Issue 44: D279-D285) is termed NUC2. The NUC2 nucleases contain the Exo-endo-phos family domain and comprise the motif [G/Y/W/F/A/H]NI[R/Q/D/E/V] (SEQ ID NO 161) corresponding to positions 369 to 372 in *Sporormia fimetaria* (SEQ ID NO 141). The nucleotides in NUC2 can be separated into at least two distinct sub-clusters, based on structural and functional similarities, which are denoted NUC2_A and NUC2_B.

NUC2_A is part of the NUC2 subgroup, containing the Exo-endo-phos family domain as well as the Conserved Protein Domain Family EEP-1. The NUC2_A subgroup comprises the polypeptides shown in SEQ ID NOs 103-107. NUC2_A nucleases are annotated as Conserved Protein Domain Family EEP-1 (domain ID cd09083, Marchler-Bauer, CDD/SPARCLE: functional classification of proteins via subfamily domain architectures, Nucleic Acids Res. 45: D200-D203, 2017). Nucleases of NUC2_A comprise the motif GR[DN][DN]G (SEQ ID NO: 162) corresponding to positions 100 to 104 in *Cadophora fastigiate* (SEQ ID NO 103).

NUC2_B is part of the NUC2 subgroup, containing the Exo-endo-phos family domain as well as the Conserved Protein Domain Family EEP-1. The NUC2_B subgroup comprises the polypeptides shown in SEQ ID Nos 108 to 141 The sub-group NUC2_B is defined with motif SDH[D/H/L]P (SEQ ID NO 163), corresponding to positions 577 to 581 of SEQ ID NO: 141. The polypeptides of the NUC2_B domain may also comprise the motif GGNI[R/Q] (SEQ ID NO 164), corresponding to positions 368 to 372 in *Sporormia fimetaria* (SEQ ID NO: 141). Nucleases belonging to the NUC2_B domain group share the above motifs, which are thus common to the DNase polypeptides in the NUC2_B cluster.

NUC3

A subgroup of DNase_NucA_NucB (Pfam domain id PF14040, Pfam version 31.0 Finn (2016). Nucleic Acids Research, Database Issue 44: D279-D285) is termed NUC3. NUC3 nucleases contain the DNase_NucA_NucB domain and comprise the motif [LV][PTA][FY][DE][VAGPH]D[CFY][WY][AT][IM]L[CYQ] (SEQ ID NO 165) corresponding to positions 24 to 35 in *Aspergillus oryzae* (SEQ ID NO 151), and/or any of the motifs GPYCK (SEQ ID NO 166) corresponding to positions 174 to 178 in *Aspergillus oryzae* (SEQ ID NO 151) or WF[QE]IT (SEQ ID NO 167) corresponding to positions 163 to 167 in *Aspergillus oryzae* (SEQ ID NO 151).

S1P1_nuclease phylogenetic tree

A phylogenetic tree was constructed of polypeptide sequences of the invention containing an S1P1 nuclease domain, as defined in PFAM (PF02265, Pfam version 31.0 Finn (2016). Nucleic Acids Research, Database Issue 44: D279-D285). The phylogenetic tree was constructed from a multiple alignment of mature polypeptide sequences containing at least one S1P1 nuclease domain. The sequences were aligned using the MUSCLE algorithm version 3.8.31 (Edgar, 2004. Nucleic Acids Research 32(5): 1792-1797).

The polypeptide comprising the S1P1 nuclease domain comprise several conserved motifs. One example is [HQ][FILVY]X[GAQS]DX[HTGSA][QVM]P[LFM]H (SEQ ID NO 168) situated in positions corresponding to positions 116 to 126 in *Trichoderma hamatum* (SEQ ID NO 150), where H, at position 116 and D at position 120 are involved in metal ion binding, and H, at position 126 is involved in substrate binding.

Another motif contained in the S1P1 nuclease domain polypeptides is G[GA]NX[VILFY]X[VLM] (SEQ ID NO 169), located at positions 133 to 139 in SEQ ID NO 150, where G at position 133 is involved in nucleoside binding.

DETERGENT EXAMPLES

Examples 1-6. Granular laundry detergent compositions designed for hand washing or top-loading washing machines.

| | 1 (wt %) | 2 (wt %) | 3 (wt %) | 4 (wt %) | 5 (wt %) | 6 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate (SiO2:Na2O at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer[1] | 0.1 | 0.2 | 0.0 | 0.0 | 0.0 | 0.0 |
| Carboxymethyl cellulose | 1 | 0.3 | 1 | 1 | 1 | 1 |
| Protease (Savinase®, 32.89 mg active/g) | 0.1 | 0.1 | 0.1 | 0.1 | | 0.1 |
| [5] First DNase as defined herein (mg active per 100g composition) | 2.0 | 3.0 | 5.0 | 1.1 | 2.2 | 0.75 |
| [5] Second DNase as defined herein (mg active per 100g composition) | 2.0 | 3.0 | 5.0 | 1.1 | 2.2 | 0.75 |
| Lipase - Lipex® (18 mg active /g) | 0.03 | 0.07 | 0.3 | 0.1 | 0.07 | 0.4 |
| [4]Amylase Stainzyme® Plus (mg active) | 3.0 | 5.0 | 3.0 | 2.2 | 6.0 | 6.0 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| MgSO4 | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

Examples 7-13. Granular laundry detergent compositions designed for front-loading automatic washing machines.

| | 7 (wt%) | 8 (wt%) | 9 (wt%) | 10 (wt%) | 11 (wt%) | 12 (wt%) | 13 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |

(continued)

| | 7 (wt%) | 8 (wt%) | 9 (wt%) | 10 (wt%) | 11 (wt%) | 12 (wt%) | 13 (wt%) |
|---|---|---|---|---|---|---|---|
| AE7 | 2.2 | 0 | 3.2 | 0 | 0 | 0 | 1 |
| C10-12 Dimethyl hydroxyethylammonium chloride | 0.75 | 0.94 | 0.98 | 0.98 | 0 | 0 | 0 |
| Crystalline layered silicate (δ-Na2Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2: 1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Protease - Purafect® (84 mg active/g) | 0.2 | 0.2 | 0.3 | 0.15 | 0.12 | 0.13 | 0.18 |
| Lipase - Lipex® (18.00 mg active/g) | 0.05 | 0.15 | 0.1 | 0 | 0 | 0 | 0.1 |
| Cellulase - CellucleanTM (15.6 mg active/g) | 0 | 0 | 0 | 0 | 0.1 | 0.1 | 0 |
| [4]Amylase Stainzyme® Plus (mg active) | 4.0 | 5.0 | 10 | 2.2 | 4.4 | 1.5 | 1.5 |
| Mannanase - Mannaway® (4mg active/g) | 0.05 | 0.1 | 0 | 0.05 | 0.1 | 0 | 0.1 |
| 5 First DNase as defined herein (mg active per 100g detergent) | 2.0 | 2.5 | 5.0 | 1.1 | 4.0 | 0.75 | 3.0 |
| [5] Second DNase as defined herein (mg active per 100g composition) | 2.0 | 3.0 | 5.0 | 1.1 | 2.2 | 0.75 | 3.0 |
| Hexosaminidase (mg active per 100g of detergent) | 3.3 | 9.2 | 12.0 | 4.7 | 3.7 | 13.2 | 3.3 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane diphosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.000 7 | 0.001 2 | 0.000 7 | 0 | 0 | 0 | 0 |

(continued)

| | 7 (wt%) | 8 (wt%) | 9 (wt%) | 10 (wt%) | 11 (wt%) | 12 (wt%) | 13 (wt%) |
|---|---|---|---|---|---|---|---|
| S-ACMC | 0.01 | 0.01 | 0 | 0.01 | 0 | 0 | 0 |
| Direct Violet 9 (active) | 0 | 0 | 0.000 1 | 0.000 1 | 0 | 0 | 0.001 |
| Sulfate/ Water & Miscellaneous | Balance | | | | | | |
| *DNase is shown as mgs of active enzyme per 100g of detergent. | | | | | | | |

Examples 14-23. Heavy Duty Liquid laundry detergent compositions

| | 14 (wt%) | 15 (wt%) | 16 (wt%) | 17 (wt%) | 18 (wt%) | 19 (wt%) | 20 (wt%) | 21 (wt%) |
|---|---|---|---|---|---|---|---|---|
| C12-15 Alkylethoxy(1.8)sulfate | 14.7 | 11.6 | 0.0 | 16.3 | 0.0 | 17.3 | 20 | 12 |
| C11.8 Alkylbenzene sulfonate | 4.3 | 11.6 | 8.3 | 7.8 | 11.7 | 7.8 | 7 | 0 |
| C16-17 Branched alkyl sulfate | 1.7 | 1.29 | 0.0 | 3.09 | 0.0 | 3.3 | 0 | 0 |
| C12-14 Alkyl -9-ethoxylate | 0.9 | 1.07 | 0.0 | 1.31 | 0.0 | 1.31 | 5 | 0 |
| C12 dimethylamine oxide | 0.6 | 0.64 | 0.0 | 1.03 | 0.0 | 1.03 | 2 | 3 |
| Citric acid | 3.5 | 0.65 | 3 | 0.66 | 2.27 | 0.67 | 1 | 0 |
| C12-18 fatty acid | 1.5 | 2.32 | 3.6 | 1.52 | 0.82 | 1.52 | 1 | 0 |
| Sodium Borate (Borax) | 2.5 | 2.46 | 1.2 | 2.53 | 0.0 | 2.53 | 0 | 1 |
| Sodium C12-14 alkyl ethoxy 3 sulfate | 0.0 | 0.0 | 2.9 | 0.0 | 3.9 | 0.0 | 0 | 14 |
| C14-15 alkyl 7-ethoxylate | 0.0 | 0.0 | 4.2 | 0.0 | 1.9 | 0.0 | 0 | 4.2 |
| C12-14 Alkyl -7-ethoxylate | 0.0 | 0.0 | 1.7 | 0.0 | 0.5 | 0.0 | 0 | 1.7 |
| Ca chloride dihydrate | 0.0 | 0.0 | 0.0 | 0.0 | 0.045 | 0.0 | 0 | 0 |
| Ca formate | 0.09 | 0.09 | 0.0 | 0.09 | 0.0 | 0.09 | 0.09 | 0 |
| A compound: bis((C2H5O)(C2H4O)n)(C H3)-N+-CxH2x-N+-(CH3)-bis((C2H5O)(C2H4O)n); n is 20 to 30; x is 3 to 8, optionally sulphated or sulphonated | 0.0 | 0.0 | 1.2 | 0.0 | 0.66 | 0.0 | 0.0 | 1.2 |
| Random graft co-polymer[1] | 0.0 | 1.46 | 0.5 | 0.0 | 0.83 | 0.0 | 0.0 | 0.5 |
| Ethoxylated Polyethylenimine [2] | 1.5 | 1.29 | 0.0 | 1.44 | 0.0 | 1.44 | 1.44 | 0.0 |
| Diethylene triamine pentaacetic acid | 0.34 | 0.64 | 0.0 | 0.34 | 0.0 | 0.34 | 0.34 | 0.0 |
| Diethylene triamine penta (methylene phosphonic acid) | 0.0 | 0.0 | 0.3 | 0.0 | 0.3 | 0.0 | 0.0 | 0.3 |
| 1-hydroxyethyidene-1,1-diphosphonic acid | 0.0 | 0.0 | 0.0 | 0.0 | 0.18 | 0.0 | 0.0 | 0.0 |
| Dihydroxybenzene-3,5-disulfonic acid disodium salt hydrate | 0.0 | 0.0 | 0.0 | 0.0 | 0.0 | 0.19 | 0.19 | 0.0 |
| Tinopal AMS-GX | 0.0 | 0.06 | 0.0 | 0.0 | 0.0 | 0.29 | 0.29 | 0.0 |
| Tinopal CBS-X | 0.2 | 0.17 | 0.0 | 0.29 | 0.0 | 0.0 | 0.0 | 0.0 |
| Tinopal TAS-X B36 | 0.0 | 0.0 | 0.0 | 0.0 | 0.091 | 0.0 | 0.0 | 0.0 |

(continued)

| | 14 (wt%) | 15 (wt%) | 16 (wt%) | 17 (wt%) | 18 (wt%) | 19 (wt%) | 20 (wt%) | 21 (wt%) |
|---|---|---|---|---|---|---|---|---|
| Amphiphilic alkoxylated grease cleaning polymer [3] | 1.28 | 1 | 0.4 | 1.93 | 0.0 | 1.93 | 1.93 | 0.4 |
| CHEC | 0.0 | 0.0 | 0.2 | 0.0 | 0.0 | 0.0 | 0.0 | 0.2 |
| Ethanol | 2 | 1.58 | 1.6 | 5.4 | 1.2 | 3.57 | 0 | 1.6 |
| Propylene Glycol | 3.9 | 3.59 | 1.3 | 4.3 | 0.0 | 3.8 | 3.8 | 1.3 |
| Diethylene glycol | 1.05 | 1.54 | 0.0 | 1.15 | 0.0 | 1.15 | 1.15 | 0.0 |
| Polyethylene glycol | 0.06 | 0.04 | 0.0 | 0.1 | 0.0 | 0.1 | 0.1 | 0.0 |
| [4]Amylase Amplify® (mg active) | 8.0 | 7.0 | 2.5 | 4.0 | 3.0 | 1.7 | 3 | 2.5 |
| [5]DNase (mg active per 100g detergent) | 3.5 | 1.5 | 1.3 | 2.5 | 0.75 | 5.0 | 1.5 | 2.5 |
| [5] Second DNase as defined herein (mg active per 100g composition) | 2.0 | 3.0 | 5.0 | 1.1 | 2.2 | 0.75 | 1.5 | 1.5 |
| Hexosaminidase (mg active per 100g of detergent) | 3.2 | 4.1 | 7.9 | 12.4 | 3.7 | 5.0 | 17.3 | 2.1 |
| Monoethanolamine | 3.05 | 2.41 | 0.4 | 1.26 | 0.31 | 1.13 | 1.13 | 0.4 |
| NaOH | 2.44 | 1.8 | 0.0 | 3.01 | 3.84 | 0.24 | 0.24 | 0.0 |
| Sodium Cumene Sulphonate | 0.0 | 0.0 | 1 | 0.0 | 0.95 | 0.0 | 0.0 | 1 |
| Sodium Formate | 0.0 | 0.11 | 0.0 | 0.09 | 0.2 | 0.12 | 0.12 | 0.0 |
| Polyethoxylated azo thiophene dye | 0.001 | 0.001 | 0.001 | 0.05 | 0.0001 | 0.0001 | 0.0001 | 0.001 |
| Water, Aesthetics (Dyes, perfumes) and Minors (Enzymes including lipase, protease, additional amylase each at 0.2% active protein, solvents, structurants) | balance | | | | | | | |

| | 22 (wt%) | 23 (wt%) |
|---|---|---|
| C13 Branched Alkyl Sulfate | 4.0 | 1.0 |
| C11.8 Alkylbenzene sulfonate | 8.5 | 1.5 |
| C15 Branched Alkyl Sulfate | 6.0 | 3.0 |
| Sodium Lauryl Sulfate | 3.70 | 0.0 |
| C12-14 Alkyl -9-ethoxylate | 8.0 | 13.0 |
| C14-15 Alkyl-7-ethoxylate | 4.0 | --- |
| C12 dimethylamine oxide | 1.0 | 0.5 |
| Citric acid | 6.8 | 0.7 |
| C12-18 fatty acid | 1.0 | 3.0 |
| Sodium Borate (Borax) | 2.0 | 0.1 |
| Ca formate | 0.2 | 0.3 |
| Ethoxylated Polyethylenimine [2] | 3.0 | 1.3 |
| Diethylene triamine pentaacetic acid | 0.0 | 0.3 |

(continued)

| | 22 (wt%) | 23 (wt%) |
|---|---|---|
| Diethylene triamine penta (methylene phosphonic acid) | 0.1 | 0.0 |
| glutamic-N,N- diacetic acid | 0.4 | --- |
| Tinopal CBS-X | 0.2 | 0.04 |
| Amphiphilic alkoxylated grease cleaning polymer [3] | 2.0 | 2.0 |
| Ethanol | 1.7 | 0.0 |
| Propylene Glycol | 2.0 | 0.3 |
| Diethylenetriamine | 0.1 | 0.0 |
| Butylated hydroxytoluene | 0.4 | --- |
| Tinogard TS | --- | 0.1 |
| [4]Amylase Amplify® (mg active) | 8.0 | 1.7 |
| [5] First DNase as defined herein (mg active per 100g detergent) | 5 | 2 |
| [5] Second DNase as defined herein (mg active per 100g composition) | 5.0 | 2.0 |
| Monoethanolamine | 3.0 | 0.0 |
| NaOH | 1.1 | 0.0 |
| Sodium Cumene Sulphonate | 1.6 | 3.0 |
| Sodium Formate | 0.2 | 0.3 |
| Polyethoxylated azo thiophene dye | 0.001 | 0.001 |
| Water, Aesthetics (Dyes, perfumes) and Minors (Enzymes including lipase, protease, additional amylase each at 0.2% active protein, solvents, structurants) | Balance | |

Examples 24-30. Unit Dose Laundry detergent compositions. Such unit dose formulations can comprise one or multiple compartments.

| | 24 (wt%) | 25 (wt%) | 26 (wt%) | 27 (wt%) | 28 (wt%) | 29 (wt%) | 30 (wt%) |
|---|---|---|---|---|---|---|---|
| Alkylbenzene sulfonic acid | 14.5 | 14.5 | 14.5 | 14.5 | 14.5 | 23 | 23 |
| C12-18 alkyl ethoxy 2.5 sulfate | 7.5 | 7.5 | 7.5 | 7.5 | 7.5 | 16 | 16 |
| C12-18 alkyl 7-ethoxylate | 13.0 | 13.0 | 13.0 | 13.0 | 13.0 | 3.1 | 3.8 |
| C14-15 alkyl 9-ethoxylate | 0 | 0 | 0 | 0 | 0 | 1 | 0 |
| Citric Acid | 0.6 | 0.6 | 0.6 | 0.6 | 0.6 | 0.9 | 0.7 |
| Fatty Acid | 14.8 | 14.8 | 14.8 | 14.8 | 14.8 | 6.5 | 6 |
| Amylase (mg active) | 6 | 12 | 8 | 2 | 10 | 2 | 2 |
| Ethoxylated Polyethylenimine[2] | 4.0 | 4.0 | 4.0 | 4.0 | 4.0 | 4.0 | 4.0 |
| Protease (Purafect Prime®, 40.6 mg active/g) | 1.4 | 2.0 | 0.9 | 1.2 | 0 | 1 | 1 |
| Cellulase (Celluclean, active protein) | 0.1 | 0.2 | 0.0 | 0.0 | 0.1 | 0 | 0 |
| [5] First DNase described herein (mg active per 100g detergent) | 3.0 | 2.0 | 1.0 | 4.0 | 2.0 | 1 | 1 |

(continued)

| | 24 (wt%) | 25 (wt%) | 26 (wt%) | 27 (wt%) | 28 (wt%) | 29 (wt%) | 30 (wt%) |
|---|---|---|---|---|---|---|---|
| [5] Second DNase as defined herein (mg active per 100g composition) | 1.0 | 2.0 | 3.0 | 1.1 | 1.2 | 0.75 | 3.0 |
| [4]Amylase Amplify® (active protein) | 0.0 | 0.0 | 0.1 | 0.2 | 0.1 | 0.5 | 0.5 |
| [6]Hexosaminidase as defined herein (mg active per 100g of detergent) | 2.2 | 3.1 | 2.3 | 5.2 | 5.3 | 12.2 | 5.4 |
| Hydroxyethane diphosphonic acid | 1.2 | 1.2 | 1.2 | 1.2 | 1.2 | 0 | 2.3 |
| Brightener | 0.3 | 0.3 | 0.3 | 0.3 | 0.3 | 0.2 | 0.2 |
| P-diol | 15.8 | 13.8 | 13.8 | 13.8 | 13.8 | 12.2 | 12.2 |
| Glycerol | 6.1 | 6.1 | 6.1 | 6.1 | 6.1 | 4.0 | 3.8 |
| MEA | 8.0 | 8.0 | 8.0 | 8.0 | 8.0 | 8.6 | 10.2 |
| TIPA | 0.0 | 0.0 | 2.0 | 0.0 | 0.0 | 0.0 | 0.0 |
| TEA | 0.0 | 2.0 | 0.0 | 0.0 | 0.0 | 0.0 | 0.0 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| Water | 10 | 10 | 10 | 10 | 10 | 10 | 10 |
| Structurant | 0.14 | 0.14 | 0.14 | 0.14 | 0.14 | 0.14 | 0.14 |
| Perfume | 1.9 | 1.9 | 1.9 | 1.9 | 1.9 | 1.9 | 1.9 |
| Buffers (monoethanolamine) | To pH 8.0 | | | | | | |
| Solvents (1,2 propanediol, ethanol) & minors | To 100% | | | | | | |

Example 31. Multiple Compartment Unit Dose Composition

Multiple compartment unit dose laundry detergent formulations of the present invention are provided below. In these examples the unit dose has three compartments, but similar compositions can be made with two, four or five compartments. The film used to encapsulate the compartments is polyvinyl alcohol.

| Base composition 1 | 31 (wt%) |
|---|---|
| Glycerol (min 99) | 5.3 |
| 1,2-propanediol | 10.0 |
| Citric Acid | 0.5 |
| Monoethanolamine | 10.0 |
| Caustic soda | - |
| Dequest 2010 | 1.1 |
| Potassium sulfite | 0.2 |
| [5] First DNase as defined herein (mg active) | 8.0 |
| [5] Second DNase as defined herein (mg active per 100g composition) | 1.0 |
| Nonionic Marlipal C24EO7 | 20.1 |

(continued)

| | |
|---|---|
| Base composition 1 | 31 (wt%) |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

Multi-compartment formulations

| Composition | 1 | | | 2 | | |
|---|---|---|---|---|---|---|
| Compartment | A | B | C | A | B | C |
| Volume of each compartment | 40 ml | 5 ml | 5 ml | 40 ml | 5 ml | 5 ml |
| Active material in Wt.% | | | | | | |
| Perfume | 1.6 | 1.6 | 1.6 | 1.6 | 1.6 | 1.6 |
| Dyes | < 0.01 | < 0.01 | < 0.01 | < 0.01 | < 0.01 | < 0.01 |
| TiO2 | 0.1 | - | - | - | 0.1 | - |
| Sodium Sulfite | 0.4 | 0.4 | 0.4 | 0.3 | 0.3 | 0.3 |
| Acusol 305 | 1.2 | | | 2 | - | - |
| Hydrogenated castor oil | 0.14 | 0.14 | 0.14 | 0.14 | 0.14 | 0.14 |
| Base Composition 1 | Add to 100% | Add to 100% | Add to 100% | Add to 100% | Add to 100% | Add to 100% |

Example 32-35. Fabric softener compositions of the present invention

| | Weight % | | | |
|---|---|---|---|---|
| | Ex. 32 | Ex. 33 | Ex. 34 | Ex. 35 |
| NaHEDP | 0.007 | 0.007 | 0.007 | 0.007 |
| Formic acid | 0.044 | 0.044 | 0.044 | - |
| HCl | - | 0.009 | 0.009 | 0.009 |
| Preservative[a] | 0.022 | 0.01 | 0.01 | 0.01 |
| FSA[b] | 7.6 | 7.6 | 7.6 | 7.6 |
| Antifoam[c] | 0.1 | 0.1 | 0.1 | 0.1 |
| coconut oil | 0.3 | 0.3 | 0.3 | 0.3 |
| isopropanol | 0.78 | 0.78 | 0.77 | 0.77 |
| Encapsulated perfume[d] | 0.15 | 0.15 | 0.15 | 0.15 |
| dye | 0.015 | 0.015 | 0.015 | 0.015 |

(continued)

| | Weight % | | | |
|---|---|---|---|---|
| | Ex. 32 | Ex. 33 | Ex. 34 | Ex. 35 |
| Cationic polymeric thickener[e] | 0.15 | 0.20 | 0.28 | 0.35 |
| [5] First DNase as defined herein (mg active per 100g detergent) | 2.0 | 1.0 | 1.0 | 0.5 |
| [5] Second DNase as defined herein (mg active per 100g detergent) | 3.0 | 1.1 | 1.2 | 0.75 |
| 50:50 Blend of alkyl dimethyl benzyl ammonium chloride and alkyl dimethyl ethylbenzyl ammonium chloride[f] | - | - | 0.4 | - |
| Succinic acid | - | - | - | 5 |
| Perfume | 1.0 | 1.0 | 1.0 | 1.0 |
| deionized water | Balance | Balance | Balance | Balance |

Raw Materials and Notes For Composition Examples 1-33

Linear alkylbenzenesulfonate having an average aliphatic carbon chain length C11-C18
C12-18 Dimethylhydroxyethyl ammonium chloride
AE3S is C12-15 alkyl ethoxy (3) sulfate
AE7 is C12-15 alcohol ethoxylate, with an average degree of ethoxylation of 7
AE9 is C12-16 alcohol ethoxylate, with an average degree of ethoxylation of 9
HSAS is a mid-branched primary alkyl sulfate with carbon chain length of about 16-17 as disclosed in US 6,020,303 and US 6,060,443
Polyacrylate MW 4500 is supplied by BASF
Carboxymethyl cellulose is Finnfix® V supplied by CP Kelco, Arnhem, Netherlands
CHEC is a cationically modified hydroxyethyl cellulose polymer.
Phosphonate chelants are, for example, diethylenetetraamine pentaacetic acid (DTPA) Hydroxyethane di phosphonate (HEDP)
Savinase®, Natalase®, Stainzyme®, Lipex®, CellucleanTM, Mannaway® and Whitezyme® are all products of Novozymes, Bagsvaerd, Denmark.
Purafect®, Purafect Prime® are products of Genencor International, Palo Alto, California, USA
Fluorescent Brightener 1 is Tinopal® AMS, Fluorescent Brightener 2 is Tinopal® CBS-X, Direct Violet 9 is Pergasol® Violet BN-Z NOBS is sodium nonanoyloxybenzenesulfonate
TAED is tetraacetylethylenediamine
S-ACMC is carboxymethylcellulose conjugated with C.I. Reactive Blue 19product name AZO-CM-CELLULOSE
Soil release agent is Repel-o-tex® PF
Acrylic Acid/Maleic Acid Copolymer is molecular weight 70,000 and acrylate:maleate ratio 70:30
EDDS is a sodium salt of ethylenediamine-N,N'-disuccinic acid, (S,S) isomer Suds suppressor agglomerate is supplied by Dow Corning, Midland, Michigan, USA
HSAS is mid-branched alkyl sulfate
Liquitint® Violet CT polymeric hueing dye, supplied by Milliken, Spartanburg, South Carolina, USA
Polyethoxylated azo thiophene dye is Violet DD™ polymeric hueing dye, supplied by Milliken, Spartanburg, South Carolina, USA.

[1] Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
[2] Polyethyleneimine (MW = 600) with 20 ethoxylate groups per -NH
[3] Amphiphilic alkoxylated polymer is a polyethylenimine (MW 600), prepared from a polymer that is derivatised to contain 24 ethoxylate groups per -NH and 16 Propoxylate groups per -NH.
[4]Amylase is shown as mgs of active enzyme per 100g of detergent.
[5]DNase in all of these examples is shown as mgs of active enzyme per 100g of detergent. DNase may comprise minor amounts of super oxide dismutase impurity.
[a] Proxel GXL, 20% aqueous dipropylene glycol solution of 1,2-benzisothiazolin-3-one, supplied by Lonza.

[b] N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.
[c] MP10®, supplied by Dow Corning, 8% activity
[d] as described in US 8,765,659, expressed as 100% encapsulated perfume oil [e] Rheovis® CDE, cationic polymeric thickener supplied by BASF
[f] N,N-dimethyl octanamide and N,N-dimethyl decanamide in about a 55:45 weight ratio, tradename Steposol® M-8-10 from the Stepan Company

## Claims

**1.** A cleaning or treatment composition comprising a first DNase, a second DNase and a cleaning or treatment adjunct.

**2.** A composition according to claim 1 wherein the first DNase and the second DNase are both endonucleases; or wherein the first DNase and the second DNase are both exonucleases; or wherein the first DNase and the second DNase comprise at least one endonuclease and at least one exonuclease.

**3.** A composition according to any preceding claim, wherein at least one DNase is bacterial, preferably obtained from *Bacillus.*

**4.** A composition according to any preceding claim wherein at least one DNase is fungal, preferably obtained from *Aspergillus, Rhizoctonia* or *Morchella.*

**5.** A composition according to any preceding claim wherein the composition comprises at least one fungal DNase and at least one bacterial DNase.

**6.** A composition according to any preceding claim wherein each of the first DNase and the second DNase is independently a NUC1_A, a NUC2_A, NUC2_B, NUC3 or S1P1 DNase.

**7.** A composition according to any preceding claim wherein the first and the second DNase comprise at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [LV][PTA][FY][DE][VAGPH]D[CFY][WY][AT][IM]L[CYQ] (SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

**8.** A composition according to any preceding claim wherein the first DNase comprises at least one motif selected from the group consisting of [F/L/Y/I]A[N/R]D[L/I/P/V][ (SEQ ID NO 158), C[D/N]T[A/R] (SEQ ID NO 159) and [D/Q][I/V]DH (SEQ ID NO 160), and wherein the second DNase comprises at least one motif selected from the group consisting of [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [LV][PTA] [FY][DE][VAGPH]D[CFY] [WY][AT][IM]L[CYQ] (SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

**9.** A composition according to any preceding claim wherein the first DNase comprises the motif [G/Y/W/F/A/H]NI[R/Q/D/E/V] (SEQ ID NO 161) and wherein the second DNase comprises at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), GR[DN][DN]G (SEQ ID NO: 162), SDH[D/H/L]P (SEQ ID NO 163), GGNI[R/Q] (SEQ ID NO 164), [LV][PTA][FY][DE][VAGPH]D[CFY][WY][AT][IM]L[CYQ] (SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

**10.** A composition according to any preceding claim wherein the first DNase comprises the motif GR[DN][DN]G (SEQ ID NO: 162), and wherein the second DNase comprises at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), SDH[D/H/L]P (SEQ ID NO 163), GGNI[R/Q] (SEQ ID NO 164), [LV]

[PTA][FY][DE][VAGPH]D[CFY][WY][AT][IM]L[CYQ] (SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

**11.** A composition according to any preceding claim wherein the first DNase comprises one or both of the motifs SDH[D/H/L]P (SEQ ID NO 163) and/or GGNI[R/Q] (SEQ ID NO 164), and wherein the second DNase comprises at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), [LV][PTA][FY][DE][VAGPH]D[CFY][WY][AT][IM]L[CYQ](SEQ ID NO 165), GPYCK (SEQ ID NO 166), WF[QE]IT (SEQ ID NO 167), [HQ][FILVY]X[GAQS]DX[HTGSA][QVM]P[LFM]H (SEQ ID NO 168) and G[GA]NX[VILFY]X[VLM] (SEQ ID NO 169).

**12.** A composition according to any preceding claim wherein the first DNase comprises at least one motif selected from the group consisting of [LV][PTA][FY][DE][VAGPH]D[CFY][WY][AT][IM]L[CYQ] (SEQ ID NO 165), GPYCK (SEQ ID NO 166) and WF[QE]IT (SEQ ID NO 167), and wherein the second DNase comprises at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [HQ][FILVY]X[GAQS]DX[HTGSA][QVM]P[LFM]H (SEQ ID NO 168) and G[GA]NX[VILFY]X[VLM] (SEQ ID NO 169).

**13.** A composition according to any preceding claim wherein the first DNase comprises one or both of the motifs [HQ][FILVY]X[GAQS]DX[HTGSA][QVM]P[LFM]H (SEQ ID NO 168) and/or G[GA]NX[VILFY]X[VLM] (SEQ ID NO 169), and wherein the second DNase comprises at least one motif selected from the group consisting of [F/L/Y/I]A[N/R]D[L/I/P/V][ (SEQ ID NO 158), C[D/N]T[A/R] (SEQ ID NO 159), [D/Q][I/V]DH (SEQ ID NO 160), [G/Y/W/F/A/H]NI[R/Q/D/E/V] (SEQ ID NO 161), GR[DN][DN]G (SEQ ID NO: 162), SDH[D/H/L]P (SEQ ID NO 163), GGNI[R/Q] (SEQ ID NO 164), [LV] [PTA] [FY] [DE] [VAGPH]D[CFY][WY] [AT] [IM]L[CYQ] (SEQ ID NO 165), GPYCK (SEQ ID NO 166) and WF[QE]IT (SEQ ID NO 167).

**14.** A method for treating a surface, preferably a fabric surface comprising: a) contacting a surface with a wash liquor comprising a first DNase and a second DNase and a cleaning or treatment adjunct; optionally rinsing and drying the surface.

**15.** A kit intended for cleaning or treatment, wherein the kit comprises a solution comprising a first DNase enzyme, a solution comprising a second DNase and optionally a cleaning or treatment adjunct.

Europäisches Patentamt
European Patent Office
Office européen des brevets

# EUROPEAN SEARCH REPORT

Application Number
EP 21 18 0900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MORALES-GARCIA ANA L. ET AL: "The Application of a Nuclease Enzyme to Clean Stubborn Soils and Odors in Laundry", JOURNAL SURFACTDETERG, vol. 23, no. 4, 19 February 2020 (2020-02-19), pages 797-807, XP055858419, ISSN: 1097-3958, DOI: 10.1002/jsde.12398 Retrieved from the Internet: URL:https://onlinelibrary.wiley.com/doi/full-xml/10.1002/jsde.12398> * the whole document * | 1-15 | INV. C12N9/22 C11D3/386 |
| X | CN 108 804 872 A (AGRICULTURAL GENOMICS INST AT SHENZHEN CHINESE ACADEMY OF AGRICULTURAL) 13 November 2018 (2018-11-13) | 1,2 | |
| Y | * claim 2; example 1 * | 1-15 | |
| X | CN 110 179 104 A (ZHONGJING FOOD CO LTD) 30 August 2019 (2019-08-30) * abstract; claim 1 * | 1,2 | |
| A | WO 2015/155350 A1 (NOVOZYMES AS [DK]) 15 October 2015 (2015-10-15) * example 14 * | 1-15 | |

TECHNICAL FIELDS SEARCHED (IPC)
C12N
C11D

2

The present search report has been drawn up for all claims

| Place of search | Date of completion of the search | Examiner |
|---|---|---|
| Munich | 3 December 2021 | Voigt-Ritzer, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 18 0900

This annex lists the patent family members relating to the patent documents cited in the above-mentioned European search report. The members are as contained in the European Patent Office EDP file on
The European Patent Office is in no way liable for these particulars which are merely given for the purpose of information.

03-12-2021

| Patent document cited in search report | Publication date | Patent family member(s) | Publication date |
| --- | --- | --- | --- |
| CN 108804872 A | 13-11-2018 | NONE | |
| CN 110179104 A | 30-08-2019 | NONE | |
| WO 2015155350 A1 | 15-10-2015 | BR 112016023188 A2 | 16-01-2018 |
| | | CA 2943029 A1 | 15-10-2015 |
| | | CN 106164236 A | 23-11-2016 |
| | | CN 112899086 A | 04-06-2021 |
| | | DK 3129457 T3 | 17-09-2018 |
| | | DK 3406697 T3 | 31-08-2020 |
| | | EP 3129457 A1 | 15-02-2017 |
| | | EP 3406697 A1 | 28-11-2018 |
| | | EP 3722406 A1 | 14-10-2020 |
| | | ES 2684606 T3 | 03-10-2018 |
| | | ES 2813337 T3 | 23-03-2021 |
| | | JP 6825911 B2 | 03-02-2021 |
| | | JP 2017512885 A | 25-05-2017 |
| | | US 2017107457 A1 | 20-04-2017 |
| | | US 2020248106 A1 | 06-08-2020 |
| | | WO 2015155350 A1 | 15-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017060475 A
- WO 2004067737 A
- WO 2015091989 A
- WO 2015091990 A
- WO 2015024739 A
- WO 2015143360 A
- US 6312936 B1
- US 5679630 A
- US 4760025 A
- DE 102006022216 A1
- WO 2015089447 A
- WO 2015089441 A
- WO 2016066756 A
- WO 2016066757 A
- WO 2016069557 A
- WO 2016069563 A
- WO 2016069569 A
- WO 2016174234 A
- WO 2017089093 A
- WO 2020156419 A
- DE 102006022224 A1
- WO 2020221578 A
- WO 2020221579 A
- WO 2020221580 A
- WO 8906270 A
- WO 05052161 A
- WO 05052146 A
- WO 07044993 A2
- WO 2014194032 A
- WO 2014194054 A
- WO 2014194117 A
- WO 2015193488 A
- WO 2016075078 A
- WO 9217577 A
- WO 0037627 A
- WO 08010925 A
- US 5352604 A
- US 6939702 B1
- US 20090217464 A
- WO 2013171241 A
- WO 2011084412 A
- WO 2013033318 A
- WO 2018228880 A
- WO 2018228881 A
- EP 3299457 A
- WO 2018209026 A
- WO 2017036901 A
- WO 2017005798 A
- US 4435307 A
- US 5648263 A
- US 5691178 A
- US 5776757 A
- US 7141403 B2
- WO 09148983 A
- WO 2017106676 A
- US 7153818 B
- WO 9700324 A
- EP 1022334 A
- US 5856164 A
- WO 9923211 A
- WO 9623873 A
- WO 0060060 A
- WO 06002643 A
- WO 2017192657 A
- WO 2011100410 A
- WO 2013003659 A
- US 6093562 A
- WO 09149130 A
- WO 10115021 A
- WO 2016091688 A
- WO 2014099523 A
- WO 2014164777 A
- WO 2009149271 A
- WO 2016180748 A
- WO 2018060216 A
- WO 9324618 A
- WO 9510602 A
- WO 9815257 A
- WO 2018191135 A
- WO 2015040159 A
- WO 2017021515 A
- WO 2017021516 A
- WO 2017021517 A
- WO 2019081515 A
- WO 2013167581 A
- WO 2015181299 A
- WO 2015181292 A
- WO 2017046232 A
- WO 2017046260 A
- WO 201837062 A
- WO 201837065 A
- WO 2019038059 A
- WO 2019162000 A
- WO 2015001017 A
- WO 2018037061 A
- WO 201837064 A
- WO 2019038060 A
- WO 2019038057 A

- WO 2018178061 A
- WO 2018184873 A
- WO 2017186936 A
- WO 2017186937 A
- WO 2017186943 A
- WO 2017207770 A
- WO 2019086520 A
- WO 2019086528 A
- WO 2019086530 A
- WO 2019086532 A
- WO 2019086521 A
- WO 2019086526 A
- WO 2020002604 A
- WO 2020002608 A
- WO 2020007863 A
- WO 2020007875 A
- WO 2020008024 A
- WO 2020070063 A
- WO 2020070249 A
- WO 2020088957 A
- WO 2020088958 A
- WO 2020207944 A
- WO 2015185689 A
- EP 1794275 A
- EP 1794276 A
- US 7208459 B2
- WO 201198355 A
- WO 201147987 A
- US 2012090102 A
- WO 2010145887 A
- WO 2006055787 A
- WO 2010142503 A
- US 20080177090 A
- WO 2011011799 A
- US 8138222 B
- WO 09118375 A
- WO 9813459 A
- US 6020303 A
- US 6060443 A
- US 8765659 B

**Non-patent literature cited in the description**

- **FINN.** *Nucleic Acids Research,* 2016, D279-D285
- **MARCHLER-BAUER.** CDD/SPARCLE: functional classification of proteins via subfamily domain architectures. *Nucleic Acids Res.,* 2017, vol. 45, D200-D203
- **DARTOIS et al.** *Biochemica et Biophysica Acta,* 1993, vol. 1131, 253-360
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **EDGAR.** *Nucleic Acids Research,* 2004, vol. 32 (5), 1792-1797